(19) Europäisches Patentamt
European Patent Office
Office européen des brevets

(11) Veröffentlichungsnummer : **0 310 552 B1**

(12) # EUROPÄISCHE PATENTSCHRIFT

(45) Veröffentlichungstag der Patentschrift : **13.05.92 Patentblatt 92/20**

(21) Anmeldenummer : **88810643.2**

(22) Anmeldetag : **21.09.88**

(51) Int. Cl.$^5$ : **G03C 7/26,** C07D 335/02, // (C07D495/10, 335:00, 317:00), (C07D495/10, 335:00, 319:00)

(54) **Stabilisatoren für farbphotographische Aufzeichnungsmaterialien.**

(30) Priorität : **30.09.87 CH 3799/87**

(43) Veröffentlichungstag der Anmeldung : **05.04.89 Patentblatt 89/14**

(45) Bekanntmachung des Hinweises auf die Patenterteilung : **13.05.92 Patentblatt 92/20**

(84) Benannte Vertragsstaaten : **BE DE FR GB IT NL**

(56) Entgegenhaltungen :
**EP-A- 0 159 912**
**EP-A- 0 226 849**
**DE-A- 3 011 279**
**US-A- 4 514 481**
**JOURNAL OF ORGANIC CHEMISTRY, Band 42, Nr. 16, 1977, Seiten 2777-2778, American Chemical Society, Easton, US; C.H. CHEN etal.: "Synthesis of 4H-thiopyran-4-ones"**
**JOURNAL OF HETEROCYCLIC CHEMISTRY, Band 15, März 1978, Seiten 289-291, Heterocorp., Provo, US; C.H. CHEN et al.: "Synthesisof 5,6-dihydro-2H-thiopyrans"**
**MACROMOLECULES, Band 17, Nr. 11, 1984, Seiten 2241-2243, American Chemical Society, Easton, US; H.E. KATZ et al.: "Utility oflewis bases in alkyltin trithiolate stabilizer systems for poly(vinyl chloride)"**
**CHEMICAL AND PHARMACEUTICAL BULLETIN, Band 33, Nr. 11, 1985, Seiten 5048-5052, Pharmaceutical Society of Japan, Tokyo, JP; K.NAGASAWA et al.: "Organosulfur chemistry. II. Use of dimenthyl sulfoxide; a facile synthesis of cyclic sulfides"**
**THE JOURNAL OF ORGANIC CHEMISTRY, Band 44, Nr. 4, 16. Februar 1979, Seiten 471-477, American Chemical Society, Easton, US; K.RAMALINGAM et al.: "Carbon-13 shifts in 1-hetera-2,6-diaryl-4-cyclohexanones and a few corresponding 1-hetera-4-cyclohexanols"**

(73) Patentinhaber : **CIBA-GEIGY AG**
**Klybeckstrasse 141**
**CH-4002 Basel (CH)**

(72) Erfinder : **Rody, Jean, Dr.**
**Rütiring 82**
**CH-4125 Riehen (CH)**
Erfinder : **Leppard, David G., Dr.**
**Route de Bourguillon 6**
**CH-1723 Marly (CH)**

**EP 0 310 552 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

(56) Entgegenhaltungen :
**JOURNAL OF ORGANIC CHEMISTRY, Band 44, Nr. 4, 1979, Seiten 477-486, American Chemical Society, Easton, US; K. RAMALINGAM etal.: "Preparation and stereochemistry of some substituted 4-thianones and 4-thianols. Single-crystal analysis ofr-2,trans-6-diphenyl-cis-3-methyl-4-thianone and r-2,trans-6-diphenyl-cis-3-ethyl-4-thianone"**
**JOURNAL OF ORGANIC CHEMISTRY, Band 46, Nr. 22, 1981, Seiten 4376-4383, American Chemical Society, Easton, US; P.K.SUBRAMANIAN et al.: "Synthesis and conformational analysis of substituted 4-aminothianes"**
**JOURNAL OF THE CHEMICAL SOCIETY-PERKIN TRANSACTIONS I, Nr. 5, 1982, Seiten 1177-1182, The Chemical Society, Letchworth, GB;R.J. BATTEN et al.: "Organocuprate conjugate addition reactions of 2,3-dihydrothiin-4-one, its oxide and dioxide"**
**JOURNAL OF THE CHEMICAL SOCIETY-PERKIN TRANSACTIONS I, Nr. 4, 1984, Seiten 703-708, The Chemical Society, Letchworth, GB;V.K. KANSAL et al.: "Substituted thian-4-ones. Part 3. Synthesis and reactions of 2-alkyl-5,6-dihydrothiin-4-ones"**
**JOURNAL OF ORGANIC CHEMISTRY, Band 52, Nr. 9, 1. Mai 1987, Seiten 1703-1710, American Chemical Society, Easton, US; H.MATSUYAMA et al.: "A regioselective synthesis of cyclopentenones from 4-thianone"**
**NOUVEAU JOURNAL DE CHIMIE, Band 1, Nr. 5, 1977, Seiten 355-356, Paris, FR; M. DVOLAITZKY et al.: "Synthesis and use of some-spin-labelled long chain quaternary ammonium salts"**
**ANGEWANDTE CHEMIE-INTERNATIONAL EDITION, Band 16, Nr. 3, 1977, Seiten 195-196, Verlag Chemie, Weinheim, DE; H.-J. GAIS:"Cyclic dithiohemiacetals-synthesis and properties"**

**Beschreibung**

Die vorliegende Erfindung betrifft die Lichtstabilisierung von photographischen Farbstoffen und Farbstoffkupplern durch Zusatz eines Stabilisators in die photographische Schicht. Der Zusatz kann in der Gelbschicht, Magentaschicht oder Cyanschicht erfolgen. Die Wirkung kann durch Zusatz eines phenolischen Antioxidans noch erhöht werden.

Die meisten heute gebräuchlichen Silber-Farbbild-Verfahren beruhen auf einer chemischen Reaktion zwischen einem Farbstoffkuppler - einer Verbindung mit einer reaktiven Methylen-oder Pseudomethylengruppe und einem oxidierten Entwickler. Der Entwickler - üblicherweise ein p-Phenylendiaminderivat - wird dabei vom photosensibilisierten Silberhalogenid oxidiert unter Bildung von Silbermetall. Der oxidierte Entwickler ist das eigentliche Reagens zur Farbstoffbildung.

Entsprechend ihren chemischen Strukturen benötigen die Farbkuppler entweder 2 oder 4 Mol Silber pro Mol Kuppler. Man spricht daher von diäquivalenten oder tetraäquivalenten Kupplern.

Farbphotographische Materialien, die auf einem solchen Prozess beruhen, haben mindestens drei farbempfindliche Schichten, die drei verschiedene Sorten von Kupplern enthalten:

(a) Die Gelbschicht, enthaltend einen Gelbkuppler. Dies sind meist β-Ketocarbonamide.

(b) Die Cyanschicht, enthaltend einen Cyankuppler. Dies sind meist Phenole oder Naphthole.

(c) Die Purpur- oder Magentaschicht, enthaltend einem Magentakuppler. Diese sind meist Pyrazolone, Pyrazoloazole, Cyanoacetylcumarone oder offenkettige Acylacetonitrile.

Um eine Diffusion der Kuppler in die Nachbarschicht zu vermeiden, versieht man die Verbindungen mit hydrophoben Ballastgruppen oder bindet sie in polymere Strukturen ein.

Die nach der Entwicklung entstandenen gelben Farbstoffe besitzen eine ungenügende Lichtechtheit. Die Lichtechtheit lässt sich zu einem gewissen Grade dadurch verbessern, dass man oberhalb der Gelbschicht eine Schicht einführt, die einen UV-Absorber enthält, wie es z.B. im US-A-3,253,921 vorgeschlagen wurde.

Ein anderer, besserer Weg ist es, der Gelbschicht spezielle Stabilisatoren zuzusetzen. Diese dürfen nicht mit dem Kuppler oder dem Entwickler reagieren. Als solche Stabilisatoren wurden verschiedene phenolische Verbindungen vorgeschalgen, insbesondere aber p-Hydroxybenzoesäureester-Derivate, wie sie z.B. in der US-A-4,228,235 vorgeschalgen wurden, und Kombinationen von sterisch gehinderten Aminen mit Phenolen, wie sie z.B. in der EP-A-114,029 vorgeschlagen wurden. Auch molekulare Kombinationen von gehinderten Aminen und phenolischen Gruppen wurden vorgeschlagen, z.B. in der EP-A-82817 und EP-A-103,540. Solche Stabilisatoren haben zu einer erheblichen Steigerung der Lichtechtheit geführt, aber man ist weiterhin an Verbesserungen der Lichtechtheit der Gelbfarbstoffe interessiert.

Auch die Magenta-Farbstoffe und -Kuppler sind nicht genügend lichtecht. Dazu kommt eine thermische Instabilität vieler Magenta-Kuppler bei der Dunkellagerung. Wie bei den Gelbfarbstoffen hat man auch für die Magenta-Farbstoffe und -Kuppler Filterschichten mit UV-Absorbern, insbesondere mit Hydroxyphenylbenztriazolen vorgeschlagen, z.B. im US-A-3,533,794, jedoch genügt dies nicht für die heutigen Anforderungen an Lichtechtheit.

Wirksam ist der Zusatz von Stabilisatoren in die Magenta-Schicht. Als solche eignen sich insbesondere Hydrochinonderivate, wie sie z.B. im US-A-3,935,016 beschrieben sind.

Auch die Cyan-Farbstoffe müssen stabilisiert werden. Dies kann ebenfalls durch UV-Absorber-Filterschichten geschehen oder durch Zusatz von speziellen Stabilisatoren zur Cyanschicht. Solche Stabilisatoren können z.B. Phenole oder gehinderte Amine sein, wie in der EP-A-113,124 beschrieben.

Gegenstand der vorliegenden Erfindung ist ein farbphotographisches Aufzeichnungsmaterial, enthaltend in mindestens einer Schicht als Stabilisator mindestens eine Tetrahydrothiopyranverbindung der Formel I,

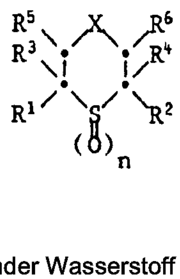

I

worin n 0, 1 oder 2 ist,
$R^1$ und $R^2$ unabhängig voneinander Wasserstoff oder Methyl bedeuten,
$R^3$ und $R^4$ unabhängig voneinander Wasserstoff, $C_1$-$C_4$-Alkyl, Phenyl, Thienyl oder durch 1 oder 2 $C_1$-$C_8$-Alkylgruppen, Cyclohexyl, Phenyl, $C_7$-$C_9$-Phenylalkyl, $C_1$-$C_{18}$-Alkoxy oder Halogen substituiertes Phenyl bedeuten,
$R^5$ und $R^6$ unabhängig voneinander Wasserstoff, $C_1$-$C_4$-Alkyl, Phenyl, -COO($C_1$-$C_{18}$-Alkyl), -CO-$CH_3$, -CO-Phenyl, -CH($OR^7$)-$CH_3$ oder -CH($OR^7$)-Phenyl bedeuten und $R^7$ Wasserstoff, $C_1$-$C_{12}$-Alkyl, $C_2$-$C_{18}$-Alkanoyl oder

Benzoyl bedeutet,
X einen zweiwertigen Rest bedeutet, der den Ring der Formel I zu einem Tetrahydrothiopyranring ergänzt und der aus einer der folgenden Gruppen besteht:

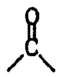 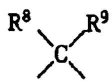 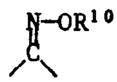 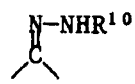 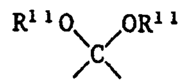

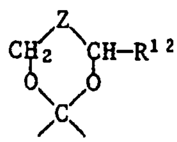 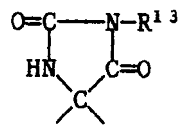 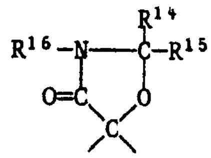

worin $R^8$ Wasserstoff, Methyl, Phenyl, -CN, $-CONH_2$, $-COO(C_1-C_4\text{-Alkyl})$ oder $-P(O)(OC_1-C_4\text{-Alkyl})_2$ bedeutet,
$R^9$ Wasserstoff, $OR^{17}$ oder $-N(R^{18})(R^{19})$ bedeutet,
$R^{10}$ Wasserstoff, $C_1$-$C_{18}$-Alkyl, $C_2$-$C_{18}$-Alkanoyl oder Benzoyl bedeutet,
$R^{11}$ $C_1$-$C_{12}$-Alkyl, Allyl oder Benzyl bedeutet,
$R^{12}$ Wasserstoff, $C_1$-$C_4$-Alkyl oder $-CH_2OR^{20}$ bedeutet,
$R^{13}$ Wasserstoff, $C_1$-$C_{18}$-Alkyl, $C_7$-$C_9$-Aralkyl, Cyclohexyl oder Phenyl bedeutet,
$R^{14}$ und $R^{15}$ unabhängig voneinander Wasserstoff, $C_1$-$C_{12}$-Alkyl oder Phenyl bedeuten oder $R^{14}$ und $R^{15}$ zusammen $C_4$-$C_{11}$-Alkylen bedeuten,
$R^{16}$ Wasserstoff, $C_1$-$C_{12}$-Alkyl, durch $-COO(C_1-C_4\text{-Alkyl})$ substituiertes $C_1$-$C_4$-Alkyl, Allyl oder Benzyl bedeutet,
$R^{17}$ Wasserstoff, $C_1$-$C_{12}$-Alkyl, Allyl, Benzyl, $C_2$-$C_{18}$-Alkanoyl, Benzoyl, eine Gruppe $-CO-O-R^{27}$ oder $-CO-NH-R^{27}$ oder eine Gruppe der Formel II oder III bedeutet,

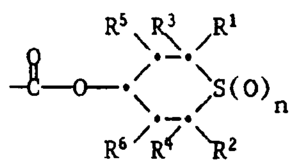 II

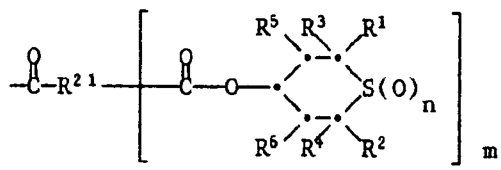 III

worin m 1, 2 oder 3 ist,
$R^{18}$ Wasserstoff, $C_1$-$C_{12}$-Alkyl, $C_2$-$C_{18}$-Alkanoyl, Benzoyl, $C_1$-$C_{12}$-Alkoxycarbonyl, Phenoxycarbonyl oder Phenylaminocarbonyl bedeutet,
$R^{19}$ Wasserstoff, $C_1$-$C_{12}$-Alkyl, Cyclohexyl, Benzyl, Phenyl oder eine Gruppe der Formel IV bedeutet,

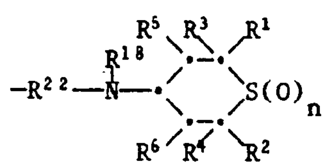 IV

$R^{20}$ Wasserstoff, $C_1$-$C_4$-Alkyl, $C_3$-$C_{18}$-Alkanoyl, Benzoyl oder eine Gruppe der Formel V oder VI

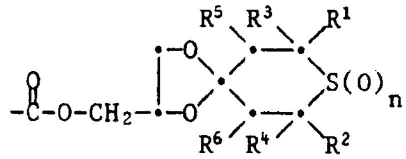

V

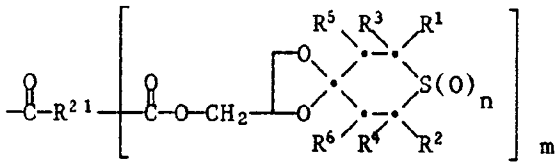

VI

bedeutet, worin m 1, 2 oder 3 ist,
$R^{21}$, wenn m 1 ist, eine direkte Bindung, $C_1$-$C_{12}$-Alkylen, durch Phenyl oder Benzyl substituiertes oder durch -O- oder -S- unterbrochenes $C_2$-$C_6$-Alkylen, Vinylen, Phenylen oder eine Gruppe -NH-$R^{28}$-NH- bedeutet,
wenn m 2 ist, $C_3$-$C_{12}$-Alkantriyl oder $C_6$-$C_{12}$-Arentriyl bedeutet, und
wenn m 3 ist, $C_4$-$C_{12}$-Alkantetrayl oder $C_6$-$C_{12}$-Arentetrayl bedeutet,
$R^{22}$ $C_2$-$C_{12}$-Alkylen, $C_4$-$C_8$-Alkenylen, Xylylen oder eine Gruppe -CO-$R^{23}$-CO-bedeutet, worin $R^{23}$ $C_1$-$C_{12}$-Alkylen, durch Phenyl oder Benzyl substituiertes oder durch -O- oder -S- unterbrochenes $C_2$-$C_6$-Alkylen, Vinylen oder Phenylen bedeutet,
Z eine direkte Bindung, eine Gruppe

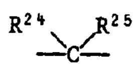

oder eine Gruppe der Formel

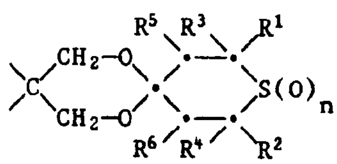

ist, worin
$R^{24}$ Wasserstoff, $C_1$-$C_4$-Alkyl, -OH oder -$CH_2OR^{26}$ bedeutet,
$R^{25}$ Wasserstoff oder $C_1$-$C_4$-Alkyl bedeutet und
$R^{26}$ Wasserstoff, $C_1$-$C_4$-Alkyl, $C_2$-$C_{18}$-Alkanoyl, Benzoyl oder eine Gruppe der Formel VII oder VIII bedeutet,

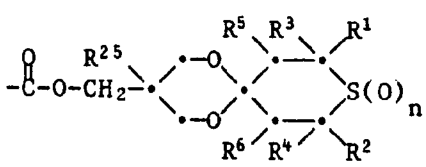

VII

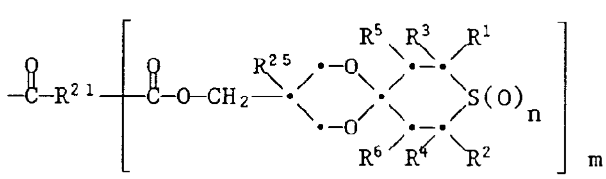

VIII

worin m 1, 2 oder 3 ist,
$R^{27}$ $C_1$-$C_{12}$-Alkyl oder Phenyl bedeutet und
$R^{28}$ $C_2$-$C_{12}$-Alkylen, $C_6$-$C_{12}$-Cycloalkylen, Phenylen, Naphthylen oder durch $C_1$-$C_4$-Alkyl, $C_1$-$C_4$-Alkoxy oder Halogen substituiertes Phenyl bedeutet.

Sofern einer der Substituenten Alkyl, Alkylen oder Alkanoyl bedeutet, kann dieser Rest unverzweigt oder verzweigt sein.

Die Verbindungen der Formel I sind z.T. bekannte Verbindungen, insbesondere die 4-Ketoverbindungen

$$(X = -\overset{O}{\overset{\|}{C}}-),$$

die z.B. in J. Org. Chem. 44, 477 (1979) beschrieben sind. Aus den 4-Ketoverbindungen erhält man durch Reduktion (z.B. mit $NaBH_4$) die 4-Hydroxyverbindungen (X = -CH(OH)-), die nach den üblichen Methoden veräthert, verestert oder carbamoyliert werden können. Durch Reduktion der entsprechenden 4-Ketoxime erhält man die 4-Aminoverbindungen, die ebenfalls nach den üblichen Methoden alkyliert, acyliert oder carbamoyliert werden können. Durch Umsetzung der 4-Ketoverbindungen mit Monoalkaholen der Formel $R^{11}OH$ erhält man die entsprechenden Dialkylketale. Durch Ketalisierung mit Diolen erhält man die cyclischen Ketale (Spiro-1,3-dioxane und Spirodioxolane). Die Spirohydantoine erhält man über die 4-Cyan-4-aminoverbindungen durch Umsetzung mit einem Isocyanat $R^{13}NCO$. Die Spirooxazolidone erhält man über die 4-Hydroxy-4-carbamoylverbindungen durch Umsetzung mit einem Aldehyd $R_{14}CH0$ oder einem Keton $R^{14}$-CO-$R^{15}$ Auch die übrigen Verbindungen der Formel I können direkt oder indirekt aus den 4-Ketoverbindungen hergestellt werden.

Weitere Verbindungen der Formel I sind aus JOURNAL OF THE CHEMICAL SOCIETY-PERKIN TRANSACTIONS I, Nr. 4, 1984, Seiten 703-708, JOURNAL OF ORGANIC CHEMISTRY, Band 52, Nr. 9, 01.05.87, Seiten 1703-1710, NOUVEAU JOURNAL DE CHIMIE, Band 1, Nr. 5, 1977, Seiten 355-356, ANGEWANDTE CHEMIE - INTERNATIONAL EDITION, Band 16, Nr. 3, 1977, Seiten 195-197, sowie Journal of American Chemical Society, 1979, 101, 5405 Davies J., Jones J.B. und Chemistry Letters, 1984, 833, Matsuyama et al bekannt. Diese Publikationen betreffen Synthese und Eigenschaften solcher Verbindungen.

Bevorzugt enthält das farbphotographische Aufzeichnungsmaterial eine Verbindung der Formel I, worin
n 0 oder 2 ist,
$R^1$ und $R^2$ Wasserstoff oder Methyl sind, $R^3$ und $R^4$ Methyl, Phenyl, Thienyl oder durch 1 oder 2 $C_1$-$C_4$-Alkylgruppen, durch Cyclohexyl, $C_1$-$C_4$-Alkoxy oder Chlor substituiertes Phenyl bedeuten,
$R^5$ und $R^6$ unabhängig voneinander Wasserstoff, Methyl, Acetyl oder Benzoyl bedeuten,
X eine der folgenden Gruppen bedeutet:

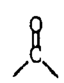 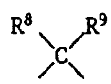 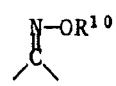 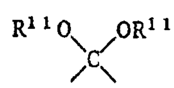 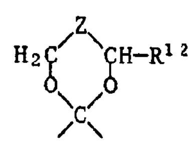

worin $R^8$ Wasserstoff, Methyl, Phenyl oder -P(O)(O$C_1$-$C_4$-Alkyl$)_2$ bedeutet,
$R^9$ Wasserstoff, -$OR^{17}$ oder -$NHR^{18}$ bedeutet,
$R^{10}$ Wasserstoff oder $C_2$-$C_{18}$-Alkanoyl ist,
$R^{11}$ $C_1$-$C_4$-Alkyl ist,
$R^{12}$ Wasserstoff, $C_1$-$C_4$-Alkyl oder -$CH_2OR^{20}$ bedeutet,
Z eine direkte Bindung oder eine Gruppe -C($R^{24}$)($R^{25}$)- bedeutet,
$R^{17}$ Wasserstoff, $C_2$-$C_{18}$-Alkanoyl, Benzoyl oder eine Gruppe der Formel III bedeutet, in der m 1 ist und
$R^{21}$ $C_1$-$C_{12}$-Alkylen, Vinylen oder Phenylen ist,
$R^{18}$ $C_2$-$C_{12}$-Alkanoyl oder Benzoyl bedeutet,
$R^{20}$ Wasserstoff $C_2$-$C_{12}$-Alkanoyl, Benzoyl oder eine Gruppe der Formel VI bedeutet, in der m 1 ist,
$R^{24}$ Wasserstoff, $C_1$-$C_4$-Alkyl oder -$CH_2OR^{26}$ bedeutet,
$R^{25}$ Wasserstoff oder $C_1$-$C_4$-Alkyl bedeutet und
$R^{26}$ Wasserstoff, $C_2$-$C_{12}$-Alkanoyl, Benzoyl oder eine Gruppe der Formel VIII bedeutet, in dem m 1 ist.

Besonders bevorzugt enthält das Aufzeichnungsmaterial eine Verbindung der Formel I, worin n 0 oder 2 ist, $R^1$ und $R^2$ Wasserstoff oder Methyl sind, $R^3$ und $R^4$ Methyl, Phenyl, Thienyl oder durch Methyl, Methoxy oder Chlor substituiertes Phenyl bedeuten, $R^5$ und $R^6$ unabhängig voneinander Wasserstoff oder Acetyl bedeuten, X eine der folgenden Gruppen bedeutet:

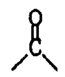 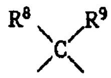 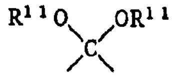 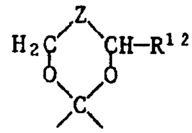

worin $R^8$ Wasserstoff, Methyl oder $-P(O)(OC_1-C_4\text{-Alkyl})_2$ bedeutet, $R^9$ $-OR^{17}$ ist, $R^{11}$ $C_1$-$C_4$-Alkyl bedeutet, $R^{12}$ Wasserstoff, $C_1$-$C_4$-Alkyl oder $-CH_2OH$ bedeutet, Z eine direkte Bindung oder eine Gruppe $-C(R^{24})(R^{25})$-ist, $R^{17}$ Wasserstoff, $C_2$-$C_{12}$-Alkanoyl, Benzoyl oder eine Gruppe der Formel III bedeutet, in der m 1 ist und $R^{21}$ $C_2$-$C_8$-Alkylen bedeutet, $R^{24}$ eine Gruppe $-CH_2OH$ und $R^{25}$ $C_1$-$C_4$-Alkyl bedeuten.

Beispiele für Verbindungen der Formel I sind:

1. 4-Oxo-2,2-dimethyl-thian
2. 4-Oxo-2,2,6,6-tetramethyl-thian
3. 4-Oxo-2,6-diphenyl-thian
4. 4-Oxo-2-phenyl-6,6-dimethyl-thian
5. 4-Oxo-2,6-bis-(4-chlorphenyl)-thian
6. 4-Oxo-2,6-bis-(4-methoxyphenyl)-thian
7. 4-Hydroxy-2,2,6,6-tetramethyl-thian
8. 4-Hydroxy-2,6-diphenyl-thian
9. 4-Hydroxy-2,6-bis-(4-chlorphenyl)-thian
10. 4-Hydroxy-2,6-bis-(4-methoxyphenyl)-thian
11. 4-Hydroxy-2,2,6,6-tetramethyl-thian-1-oxid
12. 4-Hydroxy-2,2,6,6-tetramethyl-thian-1-dioxid
13. 4-Hydroxy-2,6-diphenyl-thian-1-oxid
14. 4-Hydroxy-2,6-diphenyl-thian-1-dioxid
15. 4-Hydroxy-4-methyl-2,6-diphenyl-5-acetyl-thian
16. 4-Hydroxy-2,4,6-triphenyl-5-benzoyl-thian
17. 4-Hydroxy-2,6-dithienyl-thian
18. 4-Hydroxy-4-diäthoxyphosphonyl-2,6-diphenyl-thian
19. 4-Lauroyloxy-thian
20. 4-Acetoxy-2,2,6,6-tetramethyl-thian
21. 4-Butyroyloxy-2,2,6,6-tetramethyl-thian
22. 4-Lauroyloxy-2,2,6,6-tetramethyl-thian
23. 4-Benzoyloxy-2,2,6,6-tetramethyl-thian
24. 4-Benzoyloxy-2,2,6,6,-tetramethyl-thian-1-oxid
25. 4-Benzoyloxy-2,2,6,6-tetramethyl-thian-1-dioxid
26. Bis-(2,2,6,6-tetramethyl-4-thianyl)-oxalat
27. Bis-(2,2,6,6-tetramethyl-4-thianyl)-adipat
28. 4-Acetoxy-2,6-diphenyl-thian
29. 4-Lauroyloxy-2,6-diphenyl-thian
30. 4-Lauroyloxy-2,6-diphenyl-thian-1-oxyd
31. 4-Benzoyloxy-2,6-bis-(4-chlorphenyl)-thian
32. 4-Lauroyloxy-2,6-bis-(4-methoxyphenyl)-thian
33. Bis-(2,6-diphenyl-4-thianyl)-malonat
34. Bis-(2,6-diphenyl-4-thianyl)-succinat
35. Bis-(2,6-diphenyl-4-thianyl)-adipat
36. Bis-(2,6-diphenyl-4-thianyl)-sebacat
37. 4,4-Dimethoxy-2,6-diphenyl-thian
38. 7,7,9,9-Tetramethyl-1,4-dioxa-8-thia-spiro[4.5]-decan
39. 7,9-Diphenyl-1,4-dioxa-8-thia-spiro[4.5]-decan
40. 7,9-Diphenyl-1,4-dioxa-8-thia-spiro[4.5]-decan-8-oxyd
41. 7,9-Diphenyl-1,4-dioxa 8-thia-spiro[4.5]-decan-8-dioxyd
42. 2-Hydroxymethyl-7,9-diphenyl-1,4-dioxa-8-thia-spiro[4.5]-decan
43. 3-Ethyl-3-hydroxymethyl-8,10-diphenyl-1,5-dioxa-9-thia-spiro[5.5]-undecan
44. 3-Ethyl-3-hydroxymethyl-8,10-diphenyl-1,5-dioxa-9-thia-spiro[5.5]-undecan-9-oxyd
45. 3-Ethyl-3-hydroxymethyl-8,10-diphenyl-1,5-dioxa-9-thia-spiro[5.5]-undecan-9-dioxyd
46. 3-Ethyl-3-hydroxymethyl-8,10-bis-(4-chlorphenyl-1,5-dioxa-9-thia-spiro[5.5]-undecan

47. 2-Acetoxymethyl-7,9-diphenyl-1,4-dioxa-8-thia-spiro[4.5]-decan
48. 2-Butyroyloxymethyl-7,9-diphenyl-1,4-dioxa-8-thia-spiro[4.5]-decan
49. 2-Benzoyloxymethyl-7,9-diphenyl-1,4-dioxa-8-thia-spiro[4.5]-decan
50. 3-Ethyl-3-acetoxy-8,10-diphenyl-1,5-dioxa-9-thia-spiro[5.5]-undecan
51. 3-Ethyl-3-lauroyloxymethyl-8,10-diphenyl-1,5-dioxa-9-thia-spiro[5.5]-undecan
52. 3-Ethyl-3-benzoyloxymethyl-8,10-diphenyl-1,5-dioxa-9-thia-spiro[5.5]-undecan
53. 3-Ethyl-3-caproyloxymethyl-8,10-diphenyl-1,5-dioxa-9-thia-spiro[5.5]-undecan
54. 1,3-Diazo-8-thia-7,7,9,9-tetramethyl-2,4-dioxa-spiro[4.5]-decan
55. 3-Octyl-1,3-diaza-8-thia-7,9-diphenyl-2,4-dioxa-spiro[4.5]-decan
56. 3-Benzyl-1,3-diaza-8-thia-7,9-diphenyl-2,4-dioxa-spiro[4.5]-decan
57. 2,2,7,7,9,9-Hexamethyl-8-thia-1-oxa-3-aza-4-oxo-spiro[4.5]-decan
58. 2,2-Dimethyl-7,9-diphenyl-8-thia-1-oxa-3-aza--4-oxo-spiro[4.5]-decan
59. 4-Acetamido-2,2,6,6-tetramethyl-thian
60. A-Lauroylamido-2,6-diphenyl-thian sowie die Verbindungen der Formeln

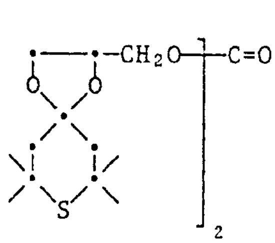

61.

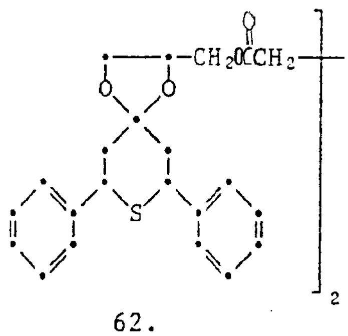

62.

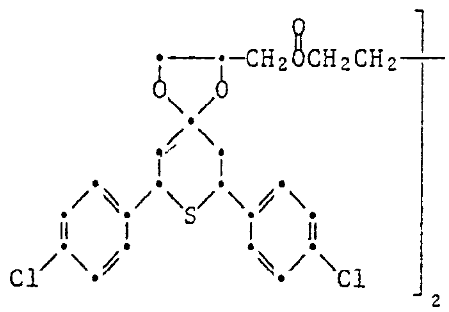

63.

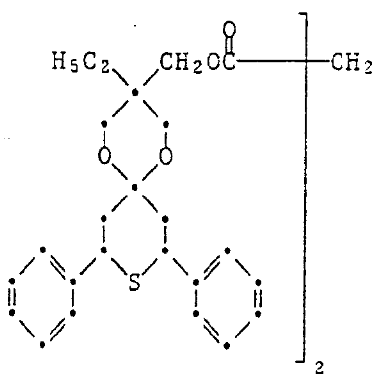

64.

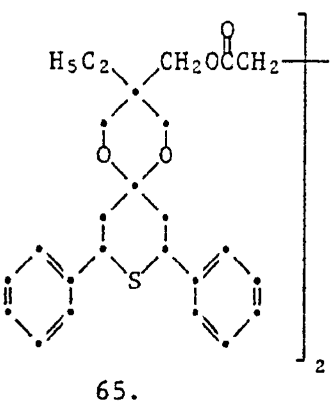

65.

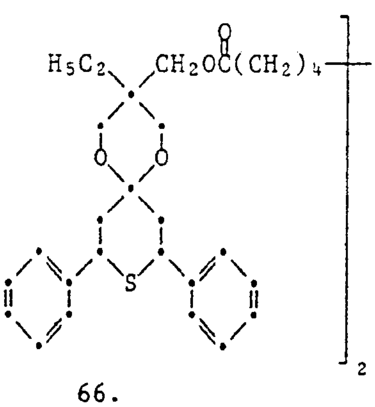

66.

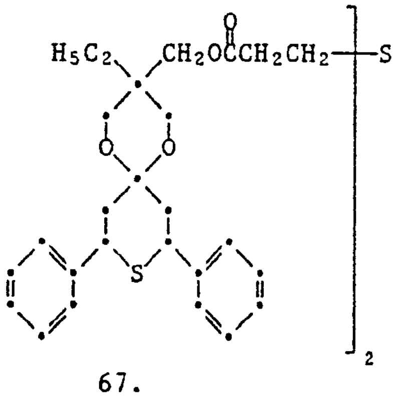

67.

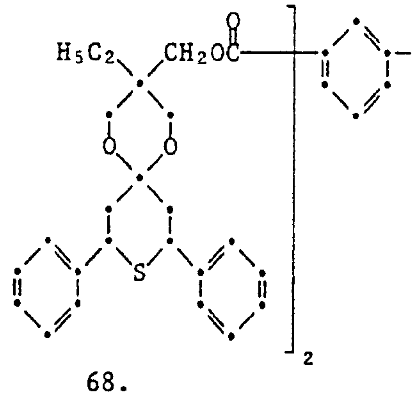

68.

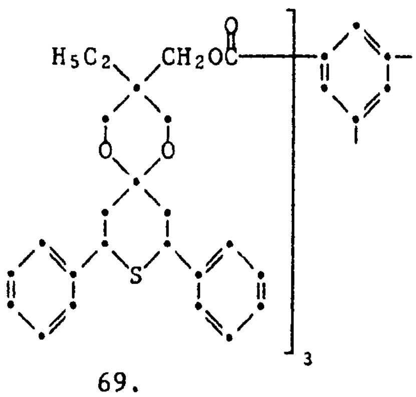

69.

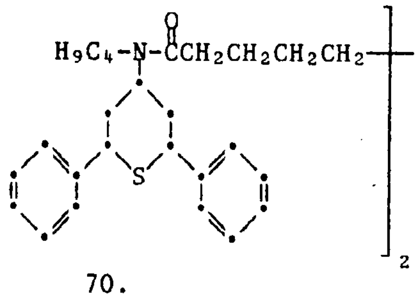

70.

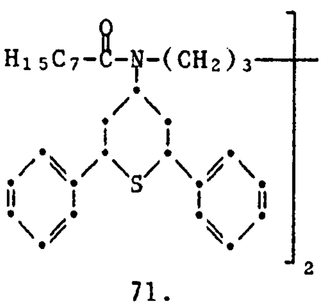

71.

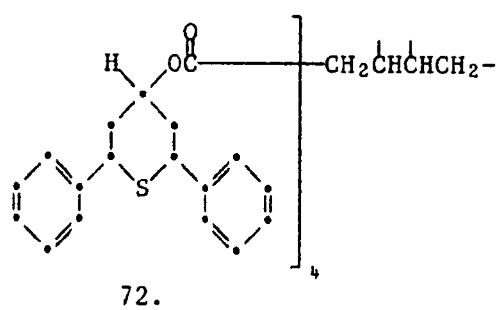

72.

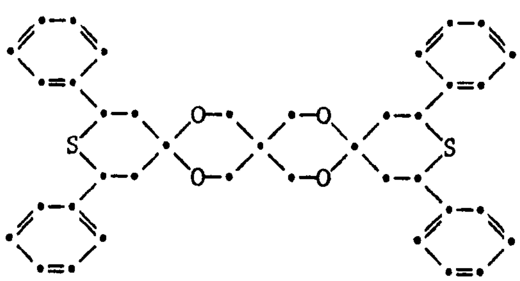

73.

Bevorzugt verwendet man die Stabilisatoren der Formeln I in Kombination mit einem phenolischen Antioxidans. Hierdurch wird nicht nur eine antioxidative Stabilisierung bewirkt, sondern überraschenderweise auch eine Steigerung der Lichtschutzwirkung. Die Erfindung betrifft daher auch ein farbphotographisches Aufzeichnungsmaterial, enthaltend

a) mindestens eine Tetrahydrothiopyranverbindung der Formel I und

b) mindestens ein phenolisches Antioxidans.

Phenolische Antioxidantien sind Verbindungen mit einer sterisch gehinderten Phenolgruppe. Die meisten dieser Verbindungen enthalten mindestens eine Gruppe der Formel

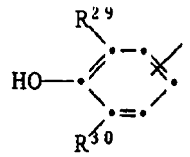

worin $R^{29}$ und $R^{30}$ unabhängig voneinander $C_1$-$C_{12}$-Alkyl, Cyclohexyl, Phenyl oder $C_7$-$C_9$-Phenylalkyl bedeuten und $R^{30}$ auch Wasserstoff bedeuten kann.

Bevorzugt sind als Komponente (b) solche Antioxidantien, die mindestens eine Gruppe der Formel

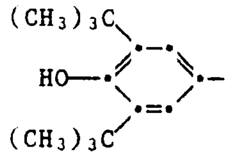

oder

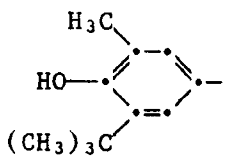

enthalten.

Das phenolische Antioxidans kann auch ein Alkylether eines sterisch gehinderten Phenols sein.

Beispiele für solche Antioxidantien, die als Komponente (b) verwendet werden können, sind die folgenden Verbindungen:

Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Di-phenyl-4-octadecyloxyphenol.

Hydroxylierte Thiodiphenylether, z.B. 2,2′-Thio-bis-(6-tert.butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

Alkyliden-Bisphenole, z.B. 2,2′-Methylen-bis-(6-tert.butyl-4-methylphenol), 2,2′-Methylen-bis-(6-tert.butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert.butylphenol), 2,2′-Ethyliden-bis-(4,6-di-tert.butylphenol), 2,2′-Ethyliden-bis-(6-tert.butyl-4-isobutylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert.butylphenol), 4,4′-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3′-tert.butyl-4′-hydroxyphenyl)-butyrat], Bis-(3-tert.butyl-4-hydroxy-5-methylphenyl)-dicyclopentadien, Bis-[2-(3′-tert.butyl-2′-hydroxy-5′-methyl-benzyl)-6-tert.butyl-4-methyl-phenyl]-terephthalat.

Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N′-Bis-(hydroxyethyl)-oxalsäurediamid.

Ester der β-(5-tert.Butyl-4-hydroxy-3-methylphenyl)-propionsäure mit ein-oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

Ester der β-(3,5-Dicyclohexyl-4-hydroxyphenyl)-propionsäure mit ein-oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

Ester der 3,5-Di-tert.butyl-4-hydroxybenzoesäure mit ein- oder mehrwertigen Alkoholen oder Phenolen wie z.B. 2,4-Di-tert. butylphenol oder 2,4-Di-tert.pentylphenol.

Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-tert.butyl-4-hydroxyphenylpropionyl)-trimethylendiamin, N,N'-Bis-(3,5-di-tert.butyl-4-hydroxyphenylpropionyl)-hydrazin.

Weitere Beispiele sind die Verbindungen der folgenden Formeln:

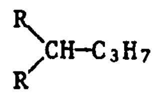

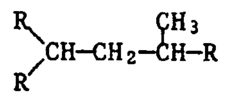

R = 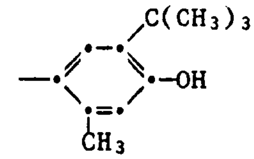

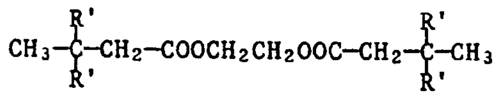

R' = 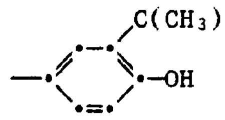

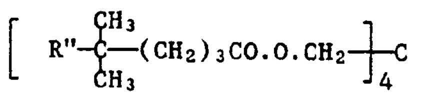

R" = 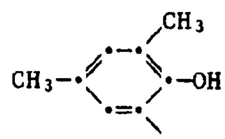

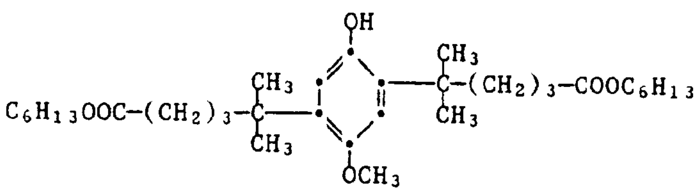

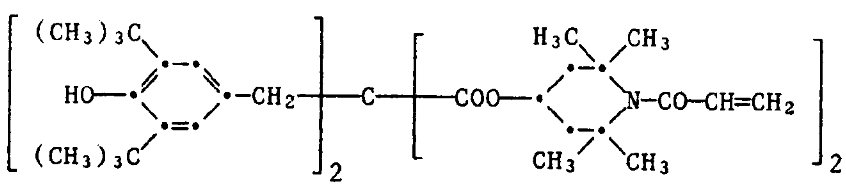

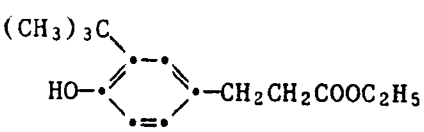

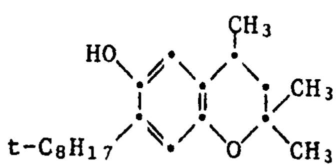

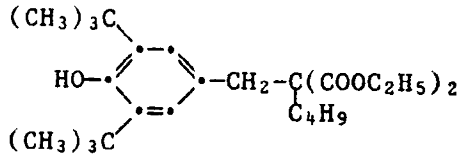

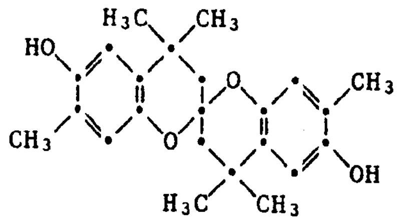

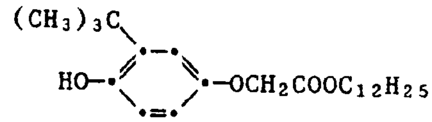

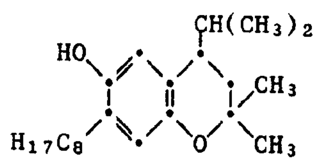

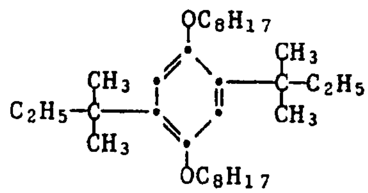

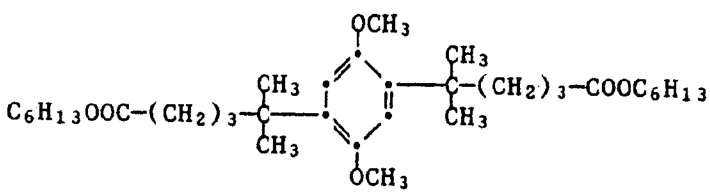

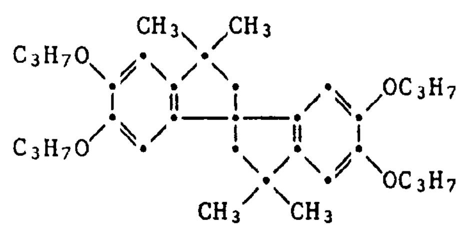

Anstelle der freien Phenole können auch verkappte Phenole verwendet werden. Beispiele hierfür sind veresterte, sulfonylierte, carbamoylierte oder silylierte Phenole. Während der Entwicklung werden daraus die freien Phenole gebildet.

Das Gewichtsverhältnis der Komponenten (a) und (b) kann in einem breiten Bereich variieren. Bevorzugt beträgt das Verhältnis (a):(b) 1:10 bis 10:1, insbesondere 1:5 bis 3:1.

Sofern die Komponenten (a) und (b) wasserlöslich sind, kann man sie als wässrige Lösung der photographischen Gelatineschicht bei deren Bereitung zusetzen. Viele dieser Verbindungen sind jedoch in Wasser nut sehr wenig löslich und man löst sie dann in einem organischen Lösungsmittel bzw. Lösungsmittelgemisch und emulgiert die Lösung in einer Gelatinelösung , die dann zur photographischen Gelatineschicht bei deren

Bereitung zugesetzt wird. Als Lösungsmittel verwendet man bevorzugt ein Gemisch eines niedrig-siedenden und eines hoch-siedenden Lösungsmittels und entfernt das niedrig-siedende Lösungsmittel während der Emulgierung. Beispiele für verwendbare niedrig-siedende Lösungsmittel sind Methylacetat, Ethylacetat, Tetrachlormethan, Dichlormethan, Trichlormethan, Methanol, Ethanol, Dioxan, Aceton oder Benzol. Beispiele für hochsiedende Lösungsmittel sind Dimethylformamid, Dimethylsulfoxid, Dialkylphthalate oder Triarylphosphate.

Das Dispergieren der Stabilisatorlösung in der Gelatinelösung kann z.B. in einer Kolloidmühle oder in einem Homogenisator oder mit Hilfe von Ultraschall geschehen. Hierbei können auch oberflächenaktive Mittel (Emulgatoren) zugesetzt werden. Eine feine Dispergierung ist Voraussetzung für die homogene Verteilung der Stabilisatoren in der photographischen Schicht.

Die Zusatzmenge an Stabilisator der Formel I beträgt pro Schicht im allgemeinen bis zu 1 g/m², vorzugsweise 10-300 mg/m². Falls eine Kombination von (a) und (b) verwendet wird, so beträgt die Zusatzmenge von (a+b) zweckmässig ebenfalls bis zu 1 g/m² und vorzugsweise 10-300 mg/m².

Der Zusatz kann zu einer oder zwei oder allen drei Farbsilberschichten erfolgen. Von besonderer Bedeutung ist der Zusatz zur Gelbschicht. In den Schichten befindet sich das sensibilisierte Silberhalogenid und der jeweilige Farbkuppler. Ausserdem können die Schichten weitere Stabilisatoren und/oder sonstige Zusätze enthalten.

Die Gelbkuppler sind vorzugsweise Verbindungen der Formel IX,

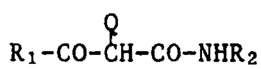

IX

worin $R_1$ Alkyl oder Aryl ist, $R_2$ Aryl ist und Q Wasserstoff oder eine Gruppe ist, die durch Reaktion mit dem oxidierten Entwickler abgespalten werden kann.

Eine Gruppe von Gelbkupplern sind solche Verbindungen der Formel IX, in denen $R_1$ tert-Butyl ist und $R_2$ eine Gruppe der Formel

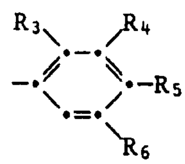

ist, worin $R_3$ Wasserstoff, Halogen, Alkyl oder Alkoxy bedeutet und $R_4$, $R_5$ und $R_6$ Wasserstoff, Halogen, Alkyl, Alkenyl, Alkoxy, Aryl, Carboxy, Alkoxycarbonyl, eine Carbamylgruppe, eine Sulfon- oder Sulfamylgruppe, eine Alkylsulfonamidogruppe, Acylaminogruppe, Ureidogruppe oder Aminogruppe bedeuten.

Vorzugsweise sind $R_3$ Chlor, $R_4$ und $R_5$ Wasserstoff und $R_6$ Acylaminogruppe. Hierzu gehören auch die Verbindungen der Formel

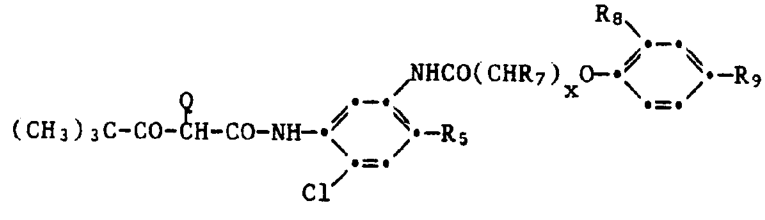

worin x 0-4 ist, $R_7$ Wasserstoff oder Alkyl ist und $R_8$ und $R_9$ Alkyl sind.

Eine andere Gruppe von Gelbkupplern entspricht der Formel X

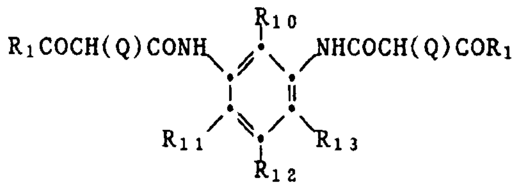

X

worin $R_{10}$ Wasserstoff, Halogen oder Alkoxy ist,
$R_{11}$, $R_{12}$ und $R_{13}$ Wasserstoff, Halogen, Alkyl, Alkenyl, Alkoxy, Aryl, Carboxyl, Alkoxycarbonyl, eine Carbamylgruppe, eine Sulfongruppe, Sulfamylgruppe, Sulfonamidogruppe, Acylaminogruppe, Ureidogruppe oder Aminogruppe bedeuten und $R_1$ und Q die oben angegebene Bedeutung haben.

Dazu gehören Verbindungen der Formel X, in denen $R_1$ tert.Butyl ist, $R_{10}$ Chlor ist, $R_{11}$ und $R_{13}$ Wasserstoff sind und $R_{12}$ Alkoxycarbonyl ist.

In den Verbindungen der Formel IX und X kann die Abgangsgruppe Q Wasserstoff oder Halogen sein oder sie ist eine heterocyclische Gruppe

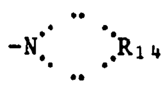

worin $R_{14}$ eine organische zweiwertige Gruppe ist, die den Ring zu einem 4-7-gliedrigen Ring ergänzt oder Q ist eine Gruppe $-OR_{15}$, worin $R_{15}$ Alkyl, Aryl, Acyl oder ein heterocyclischer Rest ist.

Typische Beispiele für gebräuchliche Gelbkuppler sind die Verbindungen der folgenden Formeln:

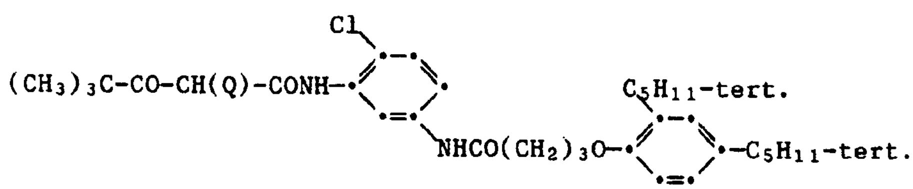

a) Q = 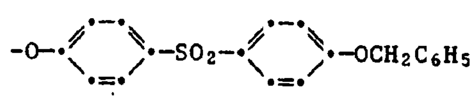

b) Q = 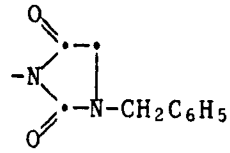

c) Q = 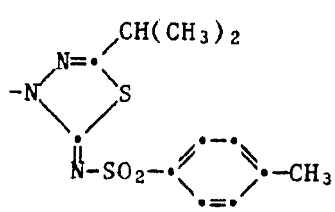

d) Q = 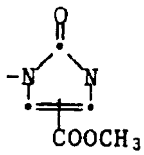

e) Q = 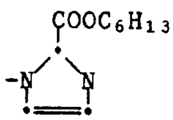

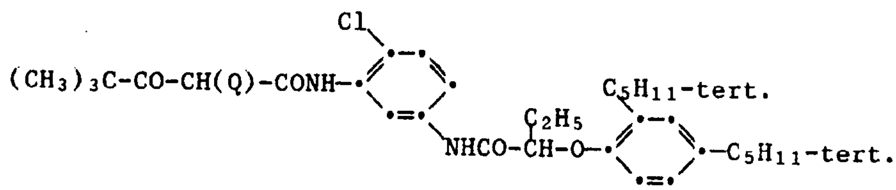

f) Q = 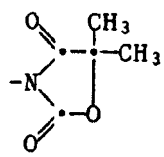

g) Q = 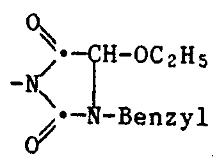

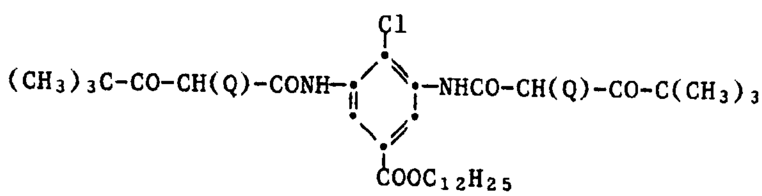

h) Q = 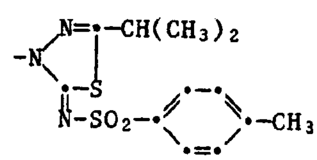

Weitere Beispiele für Gelbkuppler sind zu finden in den US-A 2,407,210, 2,778,658, 2,875,057, 2,098,513, 2,908,573, 3,227,155, 3,227,550, 2,253,924, 3,265,506, 3,277,155, 3,408,194, 3,341,331, 3,369,895, 3,384,657, 3,415,652, 3,447,928, 3,551,155, 3,582,322, 3,725,072, 3,891,445, 3,933,501,4,115,121, 4,401,752, 4,022,620, in den DE-A 1,547,868, 2,057,941, 2,162,899, 2,163,813, 2,213,461, 2,219,917, 2,261,361, 2,261,362, 2,263,875, 2,329,587, 2,414,006, 2,422,812 und in den GB-A 1,425,020 und 1,077,874.

Die Gelbkuppler werden üblicherweise in einer Menge von 0,05-2 Mol und vorzugsweise 0,1-1 Mol pro Mol Silberhalogenid verwendet.

Magenta-Kuppler können z.B. einfache 1-Aryl-5-pyrazolone sein oder mit 5-gliederigen Heteroringen kondensierte Pyrazolderivate wie z.B. Imidazopyrazole, Pyrazolopyrazole, Pyrazolotriazole oder Pyrazolotetrazole.

Eine Gruppe von Magentakupplern sind 5-Pyrazolone der Formel XI,

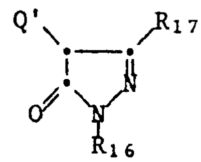

XI

wie sie in der Britischen Patentschrift 2,003,473 beschrieben sind, Darin ist $R_{16}$ Wasserstoff, Alkyl, Aryl, Alkenyl oder eine heterocyclische Gruppe, $R_{17}$ ist Wasserstoff, Alkyl, Aryl, eine heterocyclische Gruppe, eine Estergruppe, Alkoxygruppe, Alkylthiogruppe Carboxylgruppe, Arylaminogruppe, Acylaminogruppe, (Thio)harnstoffgruppe, (Thio)-carbamoylgruppe, Guanidinogruppe oder Sulfonamidogruppe.

Bevorzugt ist $R_{17}$ eine Gruppe

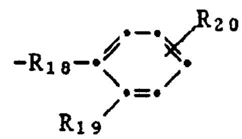

worin $R_{18}$ Imino, Acylamino oder Ureido ist, $R_{19}$ Wasserstoff, Halogen, Alkyl oder Alkoxy ist, $R_{20}$ Wasserstoff, Alkyl, Acylamino, Carbamoyl, Sulfamoyl, Sulfonamido, Alkoxycarbonyl, Acyloxy oder eine Urethangruppe ist.

Wenn Q' Wasserstoff ist, so ist der Magentakuppler tetraäquivalent in bezug auf das Silberhalogenid.

Typische Beispiele für diesen Typ von Magentakupplern sind Verbindungen der Formel

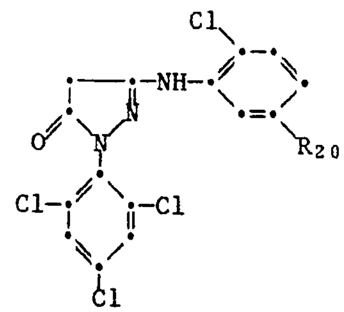

worin $R_{20}$ die oben genannten Bedeutungen hat.

Weitere Beispiele solcher tertraäquivalenter Magentakuppler sind zu finden in den US-A 2,983,608, 3,061,432, 3,062,653, 3,127,269, 3,152,896, 3,311,476, 3,419,391, 3,519,429, 3,558,319, 3,582,322, 3,615,506, 3,684,514, 3,834,908, 3,888,680, 3,891,445, 3,907,571, 3,928,044, 3,930,861, 3,930,866, 3,933,500.

Wenn Q' in Formel XI nicht Wasserstoff ist sondern eine Gruppe, die bei der Reaktion mit dem oxidierten Entwickler eliminert wird, so handelt es sich um einen diäquivalenten Magentakuppler. Q' kann in diesem Fall z.B. Halogen oder eine über O, S oder N an den Pyrazolring gebundene Gruppe sein. Solche diäquivalente Kuppler ergeben eine höhere Farbdichte und sind reaktiver gegenüber dem oxidierten Entwickler als die entsprechenden tetraäquivalenten Magentakuppler. Bevorzugt ist Q' eine O-Alkoxyarylthio-Gruppe.

Beispiele für diäquivalente Magentakuppler sind beschrieben in den US-A 3,006,579, 3,419,391, 3,311,476, 3,432,521, 3,214,437, 4,032,346, 3,701,783, 4,351,897, 3,227,554, im EP-A-133,503, DE-A-2,944,601, JP-A-78/34044, 74/53435, 74/53436, 75/53372 und 75/122935.

Ueber ein zweiwertiges Q' können 2 Pyrazolonringe verknüpft werden und man erhält dann sogenannte Bis-Kuppler. Solche sind z.B. beschrieben im US-A-2,632,702, US-A-2,618,864, GB-A-968,461, GB-A-786,859, JP-A-76/37646, 59/4086, 69/16110, 69/26589, 74/37854 und 74/29638.

Wie vorhin erwähnt können als Magentakuppler auch mit 5-gliedrigen Heterocyclen kondensierte Pyrazole - sogenannte Pyrazoloazole - verwendet werden. Deren Vorteile gegenüber einfachen Pyrazolen ist, dass sie Farben von grösserer Formalin-Beständigkeit und reineren Absorptionsspektren aufweisen.

Man kann sie durch die allgemeine Formel XII darstellen,

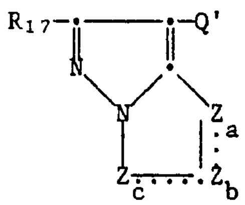

XII

worin $Z_a$, $Z_b$ und $Z_c$ die Ergänzungen zu einem 5-gliedrigen Ring bedeuten, der bis zu 4 Stickstoffatome enthalten kann. Die Verbindungen können demgemäss Pyrazolo-imidazole, Pyrazolo-pyrazole, Pyrazolo-triazole oder Pyrazolo-tetrazole sein. $R_{17}$ und Q′ haben dieselben Bedeutungen wie in Formel XI.

Pyrazolo-tetrazole sind beschrieben in der JP-A-85/33552; Pyrazolo-pyrazole in der JP-A-85/43,695; Pyrazolo-imidazole in den JP-A-85/35732, JP-A-86/18949 und US-A-4,500,630; Pyrazolo-triazole in den JP-A-85/186,567, JP-A-86/47957, JP-A-85/215,687, JP-A-85/197,688, JP-A-85/172,982, EP-A-119,860, EP-A-173, 256, EP-A-178,789, EP-A-178,788 und in Research Disclosure 84/24,624.

Weitere Pyrazoloazol-Magentakuppler sind beschrieben in: JP-A-86/28,947, JP-A-85/140,241, JP-A-85/262,160, JP-A-85/213,937, EP-A-177,765, EP-A-176,804, EP-A-170,164, EP-A-164,130, EP-A-178,794, DE-A-3,516,996, DE-A-3,508,766 und Research Disclosure 81,20919, 84/24531 und 85/25758.

Cyankuppler können z.B. Derivate von Phenol, von 1-Naphthol der von Pyrazolochinazolon sein. Bevorzugt sind Strukturen der Formel XIII,

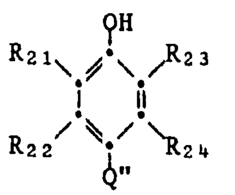

XIII

worin $R_{21}$, $R_{22}$, $R_{23}$ und $R_{24}$ Wasserstoff, Halogen,Alkyl, Carbamoyl, Amido, Sulfonamido, Phosphoramido oder Ureido sind. $R^{21}$ ist vorzugsweise H oder Cl, $R_{22}$ ist vorzugsweise eine Alkyl- oder Amidogruppe. $R_{23}$ ist vorzugsweise eine Amidogruppe und $R_{24}$ ist vorzugsweise Wasserstoff. Q″ ist Wasserstoff oder eine Abgangsgruppe, die bei der Reaktion mit dem oxidierten Entwickler abgespalten wird. Eine ausführliche Aufzählung von Cyankupplern ist in der US-A-4,456,681 zu finden.

Biespiele von gebräuchlichen Cyankupplern sind die folgenden:

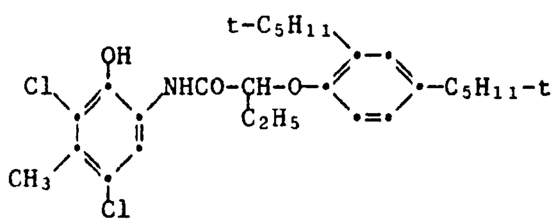

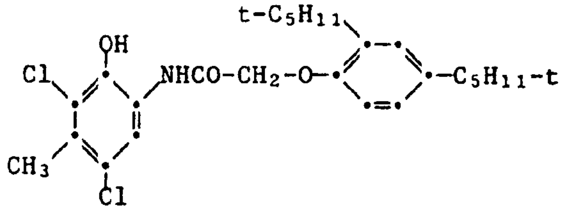

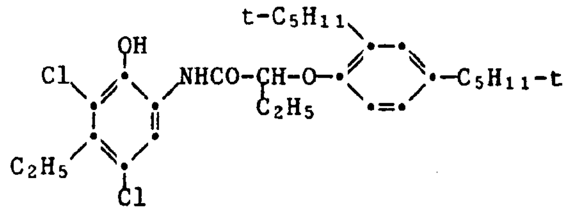

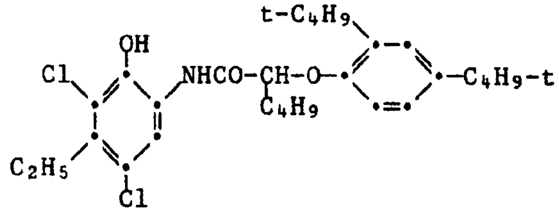

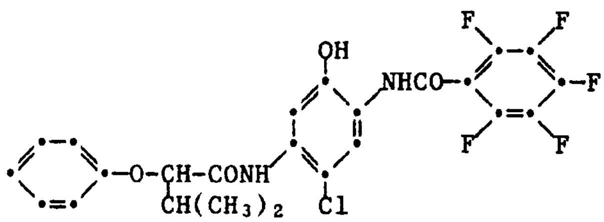

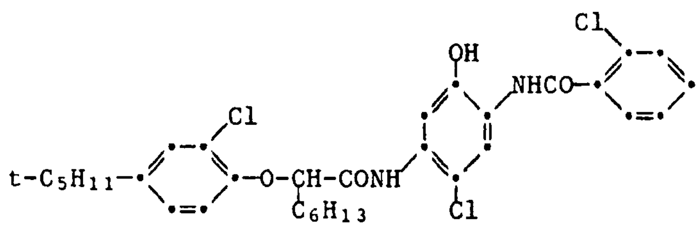

Weitere Beispiele von Cyankupplern sind in folgenden US-Patentschriften zu finden: 2,369,929, 2,423,730, 2,434,272, 2,474,293, 2,521,908, 2,698,794, 2,706,684, 2,772,162, 2,801,171, 2,895,826, 2,908,573, 3,034,892, 3,046,129, 3,227,550, 3,253,294, 3,311,476, 3,386,301, 3,419,390, 3,458,315, 3,476,560, 3,476,563, 3,516,831, 3,560,212, 3,582,322, 3,583,971, 3,591,383, 3,619,196, 3,632,347, 3,652,286, 3,737,326, 3,758,308, 3,839,044, 3,880,661, 4,004,929, 4,124,396, 4,333,999, 4,463,086, 4,456,681.

Die für farbfotographische Materialien üblicherweise verwendeten Farbentwickler sind p-Dialkylaminoaniline. Beispiele hierfür sind 4-Amino-N,N-diethylanilin, 3-Methyl-4-amino-N,N-diethylanilin, 4-Amino-N-ethyl-N-α-hydroxyethylanilin, 3-Methyl-4-amino-N-ethyl-N-α-hydroxyethylanilin, 3-Methyl-4-amino-N-ethyl-N-α-methanesulphonamidoethylanilin, 3-Methyl-4-amino-N-ethyl-N-α-methoxyethyl-anilin, 3-α-Methansulphonamidoethyl-4-amino-N,N-diethylanilin, 3-Methoxy-4-amino-N-ethyl-N-α-hydroxyethylanilin, 3-Methoxy-4-amino-N-ethyl-N-α-methoxyethylanilin, 3-Acetamido-4-amino-N,N-diethylanilin, 4-Amino-N,N-dimethylanilin, N-Ethyl-N-α-[α-(α-methoxyethoxy)ethoxy]ethyl-3-methyl-4-aminoanilin, N-Ethyl-N-α-(α-methoxyethoxy)ethyl-3-methyl-4-aminoanilin sowie die Salze solcher Verbindungen, wie z.B. Sulfate, Hydrochloride oder Toluolsulfonate.

Die erfindungsgemässen Stabilisatoren können zusammen mit dem Farbkuppler und gegebenenfalls weiteren Zusätzen in das farbphotographische Material eingearbeitet werden, indem man sie in hochsiedenden organischen Lösungsmitteln vorlöst. Vorzugsweise verwendet man Lösungsmittel, die höher als 160°C sieden. Typische Beispiele solcher Lösungsmittel sind die Ester von Phthalsäure, Phosphorsäure, Zitronensäure, Benzoesäure oder von Fettsäuren, sowie Alkylamide und Phenole.

Meist verwendet man zusätzlich noch ein niedrig siedendes Lösungsmittel, um das Einarbeiten der Zusätze in das farbphotographische Material zu erleichtern. Beispiele für solche Lösungsmittel sind Ester wie z.B. Ethylacetat, Alkohole wie z.B. Butanol, Ketone wie z.B. Methyl-isobutylketon, Chlorkohlenwasserstoffe wie z.B. Methylenchlorid, oder Amide wie z.B. Dimethylformamid. Sind die Zusätze selbst flüssig, so kann man sie auch ohne Zuhilfenahme von Lösungsmitteln in das Photomaterial einarbeiten.

Weitere Details über verwendbare hochsiedende Lösungsmittel sind in den folgenden Patentschriften zu finden.

Phosphate: GB-A-791,219, BE-A-755,248, JP-A-76/76739, 78/27449, 78/218,252, 78/97573, 79/148,113, 82/216,177, 82/92323 und 83/216,177.

Phthalate: GB-A-791,219, JP-A-77/98050, 82/93322, 82/216,176, 82/218,251, 83/24321, 83/45699, 84/79888.

Amide: GB-A-791,219, JP-A-76/105,043, 77/13600, 77/61089, 84/189,556. US-A-928,741.

Phenole: GB-A-820,329, FR-A-1,200,657, JP A-69/69946, 70/3818, 75/123,026, 75/82078, 78/17914, 78/21166, 82/212,114 und 83/45699.

Andere sauerstoffhaltige Verbindungen: US-A-748,141, 3779,765. JP-A-73/75126, 74/101,114, 74/10115, 75/101,625, 76/76740, 77/61089 und BE-A-826,039.

Sonstige Verbindungen: JP-A-72/115,369, 72/130,258, 73/127,521, 73/76592, 77/13193, 77/36294, 79/95233 und Research Disclosure 82/21918.

Die Menge von hochsiedendem Lösungsmittel liegt zweckmässig im Bereich von 0,1 bis 300 %, vorzugsweise 10 bis 100 %, bezogen auf den Farbkuppler.

Die photographischen Schichten können ferner Farbschleier-Inhibitoren enthalten. Diese verhindern das Entstehen von Farbschleiern, wie sie beispielsweise durch Reaktion des Kupplers mit unabsichtlich oxidiertem Entwickler oder mit Nebenprodukten des Farbbildungsprozesses entstehen. Solche Farbschleierinhibitoren sind meist Hydrochinonderivate, können aber auch Derivate von Aminiphenolen, von Gallussäure oder von Ascorbinsäure sein. Typische Beispiele hierfür sind in folgenden Patentschriften zu finden: US-A-2,360,290, 2,336,327, 2,403,721, 2,418,613, 2,675,314, 2,701,197, 2,704,713, 2,728,659, 2,732,300, 2,735,365; EP-A-124,877; JP-A-75/92988, 75/92989, 75/93928, 75/110,337 und 77/146,235.

Die photographischen Schichten können auch sogenannte DIR-Kuppler enthalten, die mit dem oxidierten Entwickler farblose Verbindungen ergeben. Sie werden zugesetzt zur Verbesserung der Schärfe und Körnigkeit der Farbbilder.

Die photographischen Schichten können auch UV-Absorber enthalten. Diese filtern das UV-Licht aus und schützen damit die Farbstoffe, die Kuppler oder sonstige Komponenten gegen Lichtabbau. Beispiele für solche UV-Absorber sind 2-(2-Hydroxyphenyl)-benztriazole, 2-Hydroxybenzophenone, Salicylsäureester, Acrylnitrilderivate oder Thiazoline. Solche UV-Absorber sind z.B. in folgenden Patentschriften näher aufgeführt: US-A-3,314,794, 3,352,681, 3,705,805, 3,707,375, 4,045,229, 3,700,455, 3,533,794, 3,698,907, 3,705,805, 3,738,837 und JP-A-71/2784. Bevorzugte UV-Absorber sind die 2-(2-Hydroxyphenol)-benztriazole.

Die photographischen Schichten können auch phenolische Verbindungen enthalten, die als Lichtschutzmittel für das Farbbild sowie als Mittel gegen Farbschleier wirken. Sie können in einer lichtempfindlichen Schicht (Farbschicht) oder in einer Zwischenschicht enthalten sein, allein oder zusammen mit anderen Additiven. Solche Verbindungen sind in den folgenden Patentschriften näher beschrieben: US-A-3,700,455, 3,591,381, 3,573,052, 4,030,931, 4,174,220, 4,178,184, 4,228,235, 4,279,990, 4,346,165, 4,366,226, 4,447,523, 4,528,264, 4,581,326, 4,562,146, 4,559,297, GB-A-1,309,277, 1,547,302, 2,023,862, 2,135,788, 2,139,370, 2,156,091; DE-A-2,301,060, 2,347,708, 2,526,468, 2,621,203, 3,323,448; DD-A-200,691, 214,468; EP-A-106,799, 113,124, 125,522, 159,912, 161,577, 164,030, 167,762, 176,845; JP-A-74/134,326, 76/127,730, 76/30462, 77/3822, 77/154,632, 78/10842, 79/48535, 79/70830, 79/73032, 79/147,038, 79/154,325, 79/155,836, 82/142,638, 83/224,353, 84/5246, 84/72443, 84/87456, 84/192,246, 84/192,247, 84/204,039, 84/204,040, 84/212/837, 84/220,733, 84/222,836, 84/228,249, 86/2540, 86,8843, 86/18835, 86/18836, 87/11456, 87/42245, 87/62157, 86/6652 sowie in Research Disclosure 79/17804.

Die photographischen Schichten können auch gewisse Phosphor -III-Verbindungen, insbesondere Phosphite und Phosphite, enthalten. Diese fungieren als Lichtschutzmittel für die Farbbilder sowie als Dunkellager-Stabilisator für Magentakuppler. Man setzt sie vorzugsweise den hochsiedenden Lösungsmitteln zu, zusammen mit dem Kuppler. Solche Phosphor-III-Verbindungen sind in den folgenden Patentschriften näher beschrieben: US-A-4,407,935, US-A-4,436,811, EP-A-181,289, JP-A-73/32728, JP-A-76/1420 und JP-A-55/67741.

Die photographischen Schichten können auch metallorganische Komplexe enthalten, die Lichtschutzmittel für die Farbbilder sind, insbesondere für die Magenta-Farbstoffe. Solche Verbindungen und deren Kombination mit anderen Additiven sind in folgenden Patentschriften näher beschrieben: US-A-4,050,938, 4,239,843, 4,241,154, 4,242,429, 4,241,155, 4,242,430, 4,273,854, 4,246,329, 4,271,253, 4,242,431, 4,248,949, 4,245,195, 4,268,605, 4,246,330, 4,269,926, 4,245,018, 4,301,223, 4,343,886, 4,346,165, 4,590,153; JP-A-81/167,138, 81/168,652, 82/30834, 82/161,744; EP-A-137,271, 161,577, 185,506; DE-A-2,853,865.

Die photographischen Schichten können auch Hydrochinonverbindungen enthalten. Diese wirken als

Lichtschutzmittel für die Farbkuppler und für die Farbbilder sowie als Abfänger von oxidiertem Entwickler in Zwischenschichten. Sie werden vor allem in der Magentaschicht verwendet. Solche Hydrochinon-Verbindungen und deren Kombinationen mit anderen Additiven sind in folgenden Patentschriften näher beschrieben: US-A-2,360,290, 2,336,327, 2,403,721, 2,418,613, 2,675,314, 2,701,197, 2,710,801, 2,732,300, 2,728,659, 2,735,765, 2,704,713, 2,937,086, 2,816,028, 3,582,333, 3,637,393, 3,700,453, 3,960,570, 3,935,016, 3,930,866, 4,065,435, 3,982,944, 4,232,114, 4,121,939, 4,175,968, 4,179,293, 3,591,381, 3,573,052, 4,279,990, 4,429,031, 4,346,165, 4,360,589, 4,346,167, 4,385,111, 4,416,978, 4,430,425, 4,277,558, 4,489,155, 4,504,572, 4,559,297, FR-A-885,982; GB-A-891,158, 1,156,167, 1,363,921, 2,022,274, 2,066,975, 2,071,348, 2,081,463, 2,117,526, 2,156,091; DE-A-2,408,168, 2,726,283, 2,639,930, 2,901,520, 3,308,766, 3,320,483, 3,323,699; DD-A-216,476, 214,468, 214,469, EP-A-84290, 110,214, 115,305, 124,915, 124,877, 144,288, 147,747, 178,165, 161,577; JP-A-75/33733, 75/21249, 77/128,130, 77/146,234, 79/70036, 79/133,131, 81/83742, 81/87040, 81/109,345, 83/134,628, 82/22237,82/112,749, 83/17431, 83/21249, 84/75249, 84/149,348, 84/182,785, 84/180,557, 84/189,342, 84/228,249, 84/101,650, 79/24019, 79/25823, 86/48856, 86/48857, 86/27539, 86/6652, 86/72040, 87/11455, 87/62157, sowie in Research Disclosure 79/17901, 79/17905, 79/18813, 83/22827 und 84/24014.

Die photographischen Schichten können auch Derivate von Hydrochinonethern enthalten. Diese Verbindungen wirken als Lichtschutzmittel und sind besonders geeignet zur Stabilisierung von Magenta-Farbstoffen. Solche Verbindungen und deren Kombination mit anderen Additiven sind in folgenden Patentschriften näher beschrieben:
US-A 3,285,397, 3,432,300, 3,519,429, 3,476,772, 3,591,381, 3,573,052, 3,574,627, 3,573,050, 3,698,909, 3,764,337, 3,930,866, 4,113,488, 4,015,990, 4,113,495, 4,120,723, 4,155,765, 4,159,910, 4,178,184, 4,138,259, 4,174,220, 4,148,656, 4,207,111, 4,254,216, 4,314,011, 4,273,864, 4,264,720, 4,279,990, 4,332,886, 4,436,165, 4,360,589, 4,416,978, 4,385,111, 4,459,015, 4,559,297; GB-A 1,347,556, 1,366,441, 1,547,392, 1,557,237, 2,135,788; DE-A 3,214,567; DD-214,469, EP-A 161,577, 167,762, 164,130, 176,845; JP-A 76/123,642, 77/35633, 77/147,433, 78/126, 78/10430, 78/53321, 79/24019, 79/25823, 79/48537, 79/44521, 79/56833, 79/70036, 79/7-830, 79/73032, 79/95233, 79/145,530, 80/21004, 80/50244, 80/52057, 80/70840, 80/139,383, 81/30125, 81/151,936, 82/34552, 82/68833, 82/204,036, 82/204,037,83/134,634, 83/207,039, 84/60434, 84/101,650, 84/87450, 84/149,348, 84/182,785, 86/72040, 87/11455, 87/62157, 87/63149, 86/2151, 86/6652, 86/48855 sowie in Research Disclosure 78/17051.

Das Bestreben farbphotographische Materialien in noch kürzerer Zeit zu entwickeln und dabei Chemikalien zu verwenden, die einfacher in der Handhabung und weniger umweltbelastend sind, hat zu erheblichen Beschränkungen in der Wahl der Komponenten des Systems geführt. So werden als Silberhalogenid-Emulsionen solche verwendet, die weitgehend oder ganz auf

Silberchlorid basieren, wodurch die Entwicklungszeit verkürzt wird. Weiterhin wurde gefunden, dass Entwicklersysteme weitgehend oder ganz ohne Benzylalkohol verwendet werden können, ohne dass die Farbdichte verringert wird. Dies ermöglicht Entwicklerkonzentrate aus weniger Bestandteilen, mit kürzeren Mischungszeiten und geringerer Toxizität des verbrauchten Entwicklers. Um dieses Ziel der Verkürzung der Entwicklungszeit und der Reduktion des Benzylalkohols zu erreichen, können folgende Zusätze verwendet werden:

a) N-substituierte Hydroxylamine als Antioxidantien anstelle der üblichen Hydroxylamine,

b) Entwicklungsbeschleuniger, wie z.B. 1-Aryl-3-pyrazolone, Hydrazinderivate, quartäre Ammonium- und Phosphoniumverbindungen oder Polyoxyalkylenverbindungen,

c) Triethanolamin als Teerbekämpfer,

d) Lithiumsalze, z.B. solche von Polystyrolsulfonaten,

e) aromatische Polyhydroxylverbindungen, wie z.B. 5,6-Dihydroxy-1,2,4-benzoltrisulfonsäure-Natriumsalz.

Die Verbindungen der Formel I sind auch in solchen schnellentwickelbaren Systemen brauchbar, wie in photographischen Schichten auf Basis von Silberchlorid-Emulsionen, und in Systemen, die ganz oder weitgehend ohne Benzylalkohol entwickelt werden.

Ein Teil der Verbindungen der Formel I sind neue Verbindungen. Hierzu gehören die Verbindungen der Formel Ia,

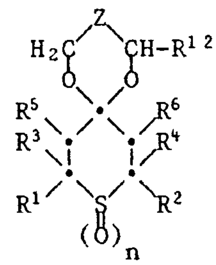

worin n, Z, $R^1$, $R^2$, $R^3$, $R^4$, $R^5$, $R^6$ und $R^{12}$ dieselben Bedeutungen haben, wie sie vorhin für Formel I angegeben wurden ausgenommen die Verbindungen der Formeln

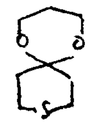 , Ia 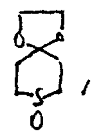 , 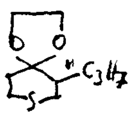 , 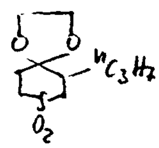 und

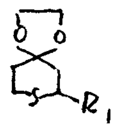 ,

worin R Wasserstoff, Methyl, Alkyl, i-Propyl, n-Butyl oder Phenyl ist. Diese Verbindungen sind ebenfalls Gegenstand der Erfindung. Sie können durch Ketalisierung der entsprechenden 4-Ketothiane der Formel XIV mit Diolen der Formel XV hergestellt wurden:

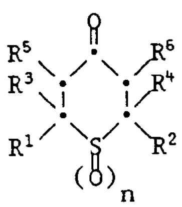 + $R^{12}$-CH(OH)-Z-$CH_2$OH ⟶ Ia

XIV XV

Die Reaktion kann nach den üblichen Methoden der Ketalisierung unter Verwendung saurer Katalysatoren ausgeführt werden. Als Katalysator kann z.B. Schwefelsäure, p-Toluolsulfonsäure oder Trifluoressigsäure verwendet werden. Vorzugsweise führt man die Reaktion in einem inerten Lösungsmittel aus, z.B. in Benzol, Toluol, Xylol oder Decalin. Das entstehende Wasser kann durch Destillation laufend aus dem Reaktionsmedium entfernt werden.

Bevorzugte Verbindungen der Formel Ia sind solche, worin n 0 oder 1 ist, $R^1$ und $R^2$ Wasserstoff oder Methyl sind, $R^3$ und $R^4$ Methyl, Phenyl, Thienyl oder durch 1 oder 2 $C_1$-$C_4$-Alkylgruppen, durch Cyclohexyl, $C_1$-$C_4$-Alkoxy oder Chlor substituiertes Phenyl bedeuten, $R^5$ und $R^6$ unabhängig voneinander Wasserstoff, Methyl, Acetyl oder Benzoyl bedeuten, $R^{12}$ Wasserstoff, $C_1$-$C_4$-Alkyl oder -$CH_2OR^{20}$ bedeutet, $R^{20}$ Wasserstoff, $C_2$-$C_{12}$-Alkanyol, Benzoyl oder eine Gruppe der Formel VI bedeutet, in der m 1 ist und $R^{21}$ $C_1$-$C_{12}$-Alkylen, Vinylen oder Phenylen ist, und Z eine direkte Bindung oder eine Gruppe -C($R^{24}$)($R^{25}$)- bedeutet, worin $R^{24}$ Wasserstoff, $C_1$-$C_4$-Alkyl oder -$CH_2OR^{26}$ bedeutet, $R^{25}$ Wasserstoff oder $C_1$-$C_4$-Alky bedeutet und $R^{26}$ Wasserstoff, $C_2$-$C_{12}$-Alkanoyl, Benzoyl oder eine Gruppe der Formel VIII bedeutet, in der m 1 ist.

Eine weitere Gruppe bevorzugter Verbindungen der Formel Ia sind jene, worin n 0 oder 2 ist, $R^1$ und $R^2$ Waserstoff oder Methyl sind, $R^3$ und $R^4$ Methyl, Phenyl, Thienyl oder durch Methyl, Methoxy oder Chlor substituiertes Phenyl bedeuten, $R^5$ und $R^6$ unabhängig voneinander Wasserstoff oder Acetyl bedeuten, $R^{12}$ Wasserstoff, $C_1$-$C_4$-Alkyl oder -$CH_2$OH bedeutet, und Z eine direkte Bindung oder eine Gruppe -C($R^{24}$)($R^{25}$)-

ist, worin $R^{24}$ eine Gruppe $-CH_2OH$ und $R^{25}$ $C_1$-$C_4$-Alkyl bedeutet.

Die folgenden Beispiele erläutern die Erfindung näher. Teile und Prozente bedeuten darin Gewichtsteile und Gewichtsprozente. Die Temperaturen sind in °C angegeben.

A) Herstellungsbeispiele für Tetrahydrothiopyrane (Thiane)

Beispiel 1:

2,7 g cis-2,6-Diphenyl-4-hydroxythian (Smp. 156°) werden mit 1,1 g Triethylamin in 50 ml absolutem Toluol vorgelegt und auf 50° erwärmt. Dazu tropft man in ca. 10 Minuten eine Lösung von 2,2 g Laurinsäurechlorid in 10 ml absolutem Toluol und rührt dann 3 Stunden bei 60°. Man lässt auf Raumtemperatur abkühlen, filtriert vom abgeschiedenen Triethylamin-hydrochlorid ab, wäscht die organische Lösung dreimal mit je 50 ml Wasser und trocknet über Natriumsulfat. Die trockene Toluollösung wird mit 50 ml Hexan verdünnt, über 40 g Kieselgel filtriert und eingedampft. Man erhält das cis-2,6-Diphenyl-4-lauroyloxythian als gelbliches Oel, das bei 50° und 1,3 Pa getrocknet wird.

Beispiel 2:

Verwendet man anstelle von 2,7 g cis-2,6-Diphenyl4-hydroxythian 3,3 g cis-2,6-Bis-(4-methoxyphenyl)-4-hydroxythian und verfährt im übrigen wie in Beispiel 1 beschrieben, so erhält man das cis-2,6-Bis-(4-methoxyphenyl)-4-lauroyloxy-thian als gelbes Oel.

Beispiel 3:

In einem 350 ml-Kolben mit KPG-Rührer, Wasserabscheider und Thermometer werden 67,1 g 2,6-Diphenyl-4-oxothian mit 35,3 g Trimethylolpropan und 2,0 g p-Toluolsulfonsäure-Monohydrat in 200 ml Toluol und 3 Stunden gekocht, bis sich kein Wasser mehr abscheidet. Der Kolbeninhalt wird auf 35-40° abgekühlt und zweimal mit je 50 ml Wasser gewaschen. Die Toluollösung wird im Vakuum auf. ca. die Hälfte eingedampft, der entstandene Kristallbrei abgenutscht und aus Toluol umkristallisiert. Man erhält das 3-Ethyl-3-hydroxymethyl-8,10-diphenyl-1,5-dioxa-9-thiaspiro[5.5]-undecan als farblose Kristalle mit einem Smp. von 128°.

Beispiel 4:

Verwendet man anstelle von 67,1 g 2,6-Diphenyl-4-oxothian 84,3 g 2,6-Bis-(4-chlorphenyl)-4-oxothian und verfährt im übrigen wie in Beispiel 3 beschrieben, so erhält man das 3-Ethyl-3-hydroxymethyl-8,10-bis-(4-chlorphenyl)-1,5-dioxa-9-thiaspiro[5.5]-undecan als farblose Kristalle mit einem Smp. von 138°.

Beispiel 5:

Verwendet man anstelle von 35,3 g Trimethylolpropan 25 g Glycerin und verfährt im übrigen wie in Beispiel 3 beschrieben, so erhält man das 2-Hydroxymethyl-7,9-diphenyl-1,4-dioxa-8-thiaspiro[4.5]-decan als farblose Kristalle mit einem Smp. von 116-118°.

Beispiel 6:

13,4 g 2,6-Diphenyl-tetrahydrothiopyran-4-on werden in 25 ml tert.-Butylamin eingetragen. Dazu tropft man bei Raumtemperatur in ca. 5 Minuten 15,2 g Diethylphosphit. Es ist eine schwache Exothermie festzustellen, die Lösung färbt sich orange und es bildet sich langsam ein Niederschlag. Man lässt 2 Stunden bei Raumtemperatur ausreagieren, filtriert den gebildeten Niederschlag ab und wäscht ihn mit 10 ml tert.-Butylamin. Der farblose Rückstand wird aus Toluol umkristallisiert. Man erhält das 2,6-Diphenyl-4-hydroxy-4-diethoxyphosphonylthian als farblose Kristalle vom Smp. 191-192°.

Beispiel 7:

Analog Beispiel 3 erhält man bei Umsetzung von 75 g 2,6-Diphenyl-4-oxothian-1-dioxid mit 35,3 g Trimethylolpropan das 3-Ethyl-3-hydroxymethyl-8,10-diphenyl-1,5-dioxa-9-thiaspiro[5.5]-undecan-9-dioxid als farblose Kristalle, die sich bei 257°C zersetzen.

Beispiel 8:

19,2 g 3-Ethyl-3-hydroxymethyl-8,10-diphenyl-1,5-dioxa-9-thispiro[5.5]-undecan und 6,8 g Methylbenzoat werden in 100 ml Xylol gelöst. Unter einem schwachen Stickstoffatom werden ca. 20 ml Xylol abdestilliert. Nach Abkühlung auf 110° gibt man 0,1 g $LiNH_2$ zu und erwärmt unter Rühren 8 Std. auf 135° unter laufendem Abdestillieren des gebildeten Methanols zusammen mit etwas Xylol. Nach Abkühlen auf Raumtemperatur werden 0,25 ml Eisessig und 3 g Bleicherde zugesetzt. Nach 15 min. Rühren wird filtriert. Das Filtrat wird über 100 g Kieselgel filtriert und im Vakuum eingedampft. Der ölige Rückstand wird bei 70° im Vakuum getrocknet. Man erhält das 3-Ethyl-3-benzoyloxymethyl-8,10-diphenyl-1,5-dioxa-9-thiaspiro[5.5]-undecan als schwach gelbliches Harz.

Beispiel 9:

Verwendet man anstelle des Methylbenzoates 6,5 g Methylcapronat und verfährt im übrigen wie in Beispiel 8 beschrieben, so erhält man das 3-Ethyl-3-caproyloxymethyl-8,10-diphenyl-1,5-dioxa-9-thiaspiro[5.5]-undecan als gelbliches Harz.

Beispiel 10:

Verwendet man anstelle des Methylbenzoates 10,7 g Methyllaurat und verfährt im übrigen wie in Beispiel 8 beschrieben, so erhält man das 3-Ethyl-3-lauroyloxymethyl-8,10-diphenyl-1,5-dioxa-9-thiaspiro[5.5]-undecan als gelbliches Harz.

Beispiel 11:

Analog Beispiel 8 erhält man aus 23,3 g 3-Ethyl-3-hydroxymethyl-8,10-diphenyl-1,5-dioxa-9-thiaspiro[5.5]-undecan und 2,2 g Dimethylsuccinat die Verbindung der Formel

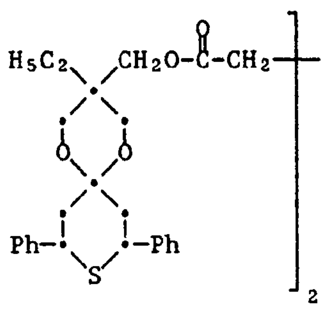

als gelbliches Harz.

Beispiel 12:

Verwendet man statt des Dimethylsuccinates 3,5 g Dimethylsebacat und arbeitet im übrigen wie in Beispiel 11, so erhält man die Verbindung der Formel

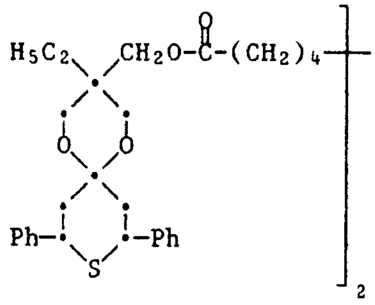

als gelbliches Harz.

Beispiel 13:

Analog Beispiel 3 werden 26,8 g 2,6-Diphenyl-4-oxothian mit 6,8 g Pentaerythrit umgesetzt. Man erhält die Verbindung der Formel

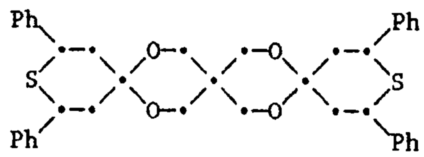

als farblose Kristalle, die bei 254-256° schmelzen.

Beispiel 14:

Analog Beispiel 3 erhält man durch Umsetzung von 53,7 g 2,6-Diphenyl-4-oxothian mit 12,4 g Ethylenglykol das 7,9-Diphenyl-1,4-dioxa-8-thiaspiro[4.5]-decan als farblose Kristalle, die bei 131° schmelzen.

Beispiel 15:

23,4 g 7,9-Diphenyl-1,4-dioxa-8-thiaspiro[4.5]-decan werden in 50 ml Methylenchlorid gelöst und die Lösung auf -10° gekühlt. Dazu tropft man innerhalb von ca. 7 Stunden eine Lösung von 16,0 g m-Chlorperbezoesäure in 200 ml Methylenchlorid, wobei darauf geachtet wird, dass die Temperatur -5° nicht übersteigt. Anschliessend wird das Reaktionsgemisch 16 Stunden bei 0° gerührt, filtriert und der Rückstand mit 100 ml Methylenchlorid gewaschen. Die vereinigten Methylenchloridlösungen werden dreimal mit je 150 ml Wasser gewaschen, über Natriumsulfat getrocknet und eingedampft. Der farblose Rückstand wird 1 Stunde mit 100 ml 2n Natronlauge gerührt, filtriert, neutral gewaschen, getrocknet und aus Toluol kristallisiert. Man erhält das 7,9-Diphenyl-1,4-dioxa-8-thiaspiro[4.5]-decan-8-oxid als farblose Kristalle mit einem Smp. von 230-32°.

Beispiel 16:

6,6 g 7,9-Diphenyl-1,4-dioxa-8-thiaspiro[4.5]-decan-8-oxid werden in 50 ml Eisessig gelöst. Zu dieser Lösung tropft man bei Raumtemperatur 10 ml Wasserstoffperoxid à 30 % in ca. 10 Minuten und rührt anschliessend 2 Stunden bei 50°. Das Reaktionsgemisch wird langsam mit 100 ml Wasser verdünnt, der entstandene Niederschlag abgenutscht, mit Wasser gewaschen und getrocknet. Durch Kristallisation aus Toluol erhält man 7,9-Diphenyl-1,4-dioxa-8-thiaspiro[4.5]-decan-8-dioxid als farblose Kristalle mit einem Smp. von 264°.

B) Anwendungsbeispiele

Beispiel I:

0,087 g des Gelbkupplers der Formel

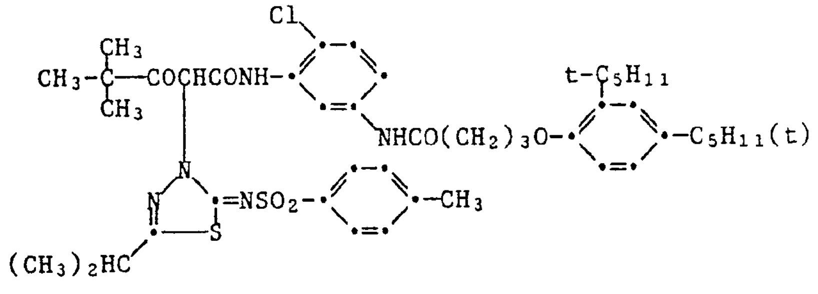

und die in der Tabelle angegebenen Mengen an Lichtschutzmittel(gemisch) werden in 2,0 ml eines Gemisches

von Trikresylphosplat/Aethylacetat (1,5 g in 100 ml) gelöst. Zu 1 ml dieser Lösung gibt man 9 ml einer 2,3 %igen Gelatinelösung , die auf einen pH-Wert von 6,5 eingestellt ist und 1,744 g/l Nekal BX®(Diisobutylnaphthalin-sulfonsäure-Na-salz) enthält.

Danach emulgiert man mit Ultraschall bei einer Leistung von 1000 Watt während 3 Minuten.

Zu 5 ml der so erhaltenen Emulsion gibt man 2 ml einer Silberbromid-Emulsion mit einem Silbergehalt von 6,0 g pro Liter und 1 ml einer 0,7%igen wässrigen Lösung von Cyanursäuredichlorid als Gelatine-Härter und giesst das Gemisch auf ein auf eine Glasplatte aufgezogenes substituiertes, kunststoffbeschichtetes, weisses Papier von 13x18 cm.

Nach dem Erstarren wird in einem Trockenschrank mit Umluft bei Raumtemperatur getrocknet. Nach 7 Tagen werden auf 35x180 mm geschnittene Proben hinter einem Stufenkeil mit 120 Lux·s belichtet und anschliessend im Ektaprint 2®-Prozess der Firma Kodak entwickelt.

Die so erhaltenen Gelbkeile werden

a) in einem Atlas Weather-Ometer® mit einer 2500 W-Xenonlampe mit total 30 kJ/cm$^2$ betrahlt.

b) in einem Atlas Weather-Omete® hinter einem UV-Filter (Kodak 2C) mit einer 2500 W-Xenonlampe mit total 60 kJ/cm$^2$ bestrahlt.

In den folgenden Tabellen 1-4 ist der prozentuale Farbdichteverlust bei einer ursprünglichen Dichte von 1,0 angegeben.

Hierbei werden die folgenden Stabilisatoren verwendet:

Thian A = 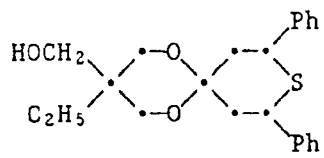 Ph = Phenyl

Thian B = 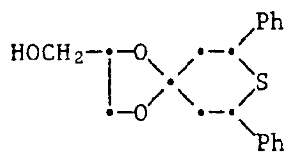

Thian C = 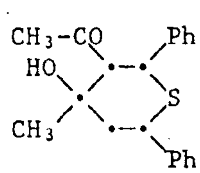

Thian D = 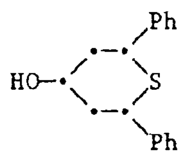

Thian E = 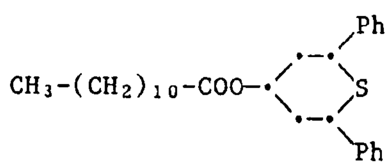

Phenol 1 = 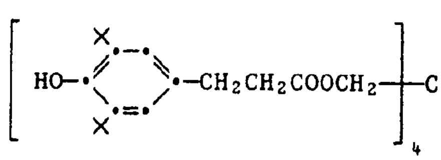 + = tert.Butyl

Phenol 2 = 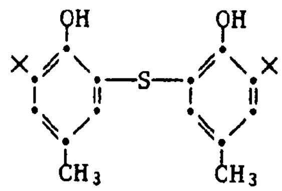

Phenol 3 = 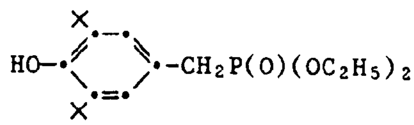

Phenol 4 = 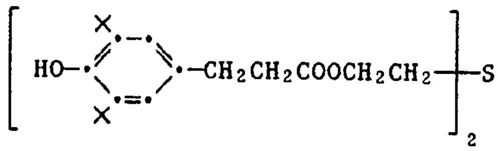

Phenol 5 = 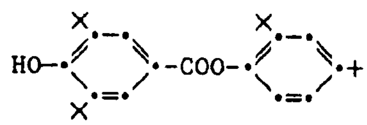

Phenol 6 = 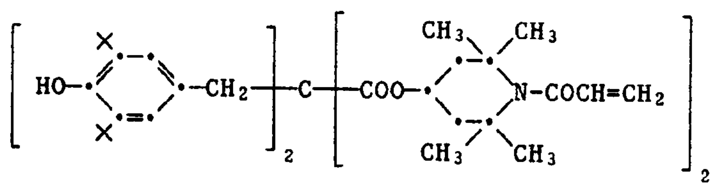

Tabelle 1

| Lichtschutzmittel | Menge (g) | Dichteverlust in % (Remission) 30 kJ/cm² | 60 kJ/cm² (UV-Filter) |
|---|---|---|---|
| Keines | - | 61 | 22 |
| Thian A | 0,026 | 58 | 14 |
| Thian A + Phenol 1 | 0,013 / 0,013 | 42 | 11 |
| Thian A | 0,026 | 58 | 14 |
| Thian A + Phenol 2 | 0,013 / 0,013 | 50 | 14 |
| Thian A | 0,026 | 58 | 14 |
| Thian A + Phenol 3 | 0,013 / 0,013 | 41 | 13 |
| Thian A | 0,026 | 58 | 14 |
| Thian A + Phenol 4 | 0,013 / 0,013 | 32 | 10 |
| Thian A | 0,026 | 58 | 14 |
| Thian A + Phenol 6 | 0,009 / 0,013 | 41 | 11 |

Tabelle 2

| Lichtschutzmittel | Menge (g) | Dichteverlust in % (Remission) 60 kJ/cm² (UV-Filter) |
|---|---|---|
| Keines | - | 25 |
| Thian B | 0,026 | 19 |
| Thian B + Phenol 1 | 0,013 / 0,013 | 7 |
| Thian B | 0,026 | 17 |
| Thian B + Phenol 5 | 0,013 / 0,013 | 11 |
| Thian A | 0,026 | 11 |
| Thian A + Phenol 5 | 0,013 / 0,013 | 8 |

Tabelle 3

| Lichtschutzmittel | Menge (g) | Dichteverlust in % (Remission) 60 kJ/cm² (UV-Filter) |
|---|---|---|
| Keines | - | 32 |
| Thian C | 0,026 | 25 |
| Thian C<br>+ Phenol 1 | 0,013<br>0,013 | 11 |
| Thian C | 0,026 | 25 |
| Thian C<br>+ Phenol 2 | 0,013<br>0,013 | 21 |

Tabelle 4

| Lichtschutzmittel | Menge (g) | Dichteverlust in % (Remission) 60 kJ/cm² (UV-Filter) |
|---|---|---|
| Keines | - | 25 |
| Thian D | 0,026 | 19 |
| Thian D<br>+ Phenol 5 | 0,013<br>0,013 | 11 |
| Thian D | 0,026 | 19 |
| Thian D<br>+ Phenol 1 | 0,013<br>0,013 | 9 |
| Thian E | 0,026 | 15 |
| Thian E<br>+ Phenol 5 | 0,013<br>0,013 | 11 |
| Thian E | 0,026 | 15 |
| Thian E<br>+ Phenol 1 | 0,013<br>0,013 | 9 |

Beispiel II:

0,025 g des Cyan-Kupplers der Formel

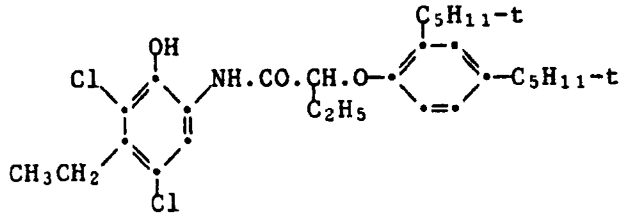

und 0,025 g eines in Tabelle 5 angegebenen Lichtschutzmittels werden in 2 ml eines Gemisches von Dibutylphthalat/Aethylacetat (0,8 g/100 ml) gelöst. Zu 1 ml dieser Lösung gibt man 9 ml einer 2,3%igen wässrigen Gelatinelösung, die auf einen pH-Wert von 6,5 eingestellt ist und 0,872 g/l Netzmittel Nekal BX® (Diisobutylnaphthalin-sulfonsäure-Na-salz) enthält. Danach emulgiert man mit Ultraschall 3 Minuten.

Zu 5 ml der so erhaltenen Kuppleremulsion gibt man 2 ml einer Silberbromid-Emulsion mit einem Silbergehalt von 3 g pro Liter und 1 ml einer 0,7%igen wässrigen Lösung von Cyanursäuredichlorid als Gelatinehärter und vergiesst es auf ein 13 x 18 cm kunststoffbeschichtetes Papier. Nach einer Härtungszeit von 7 Tagen werden die Proben durch einen Silber-Stufenkeil mit 125 Lux·s belichtet und anschliessend im Ektaprint 2®-Prozess der Firma Kodak verarbeitet.

Die so erhaltenen Farbstufenkeile werden in einem Klimaschrank 28 Tage bei 75°C und 60 % rel. Luftfeuchtigkeit gelagert.

In der folgenden Tabelle sind die dabei resultierenden prozentualen Farbdichteabnahmen bei einer ursprünglichen Farbdichte von 1,0 enthalten.

Tabelle 5

| Stabilisator | Dichteverlust in % nach 28 Tagen bei 75°C |
|---|---|
| Keine | 12 |
| Thian A | 6 |
| Thian B | 6 |

Beispiel III:

0,025 g des Cyan-Kupplers der Formel

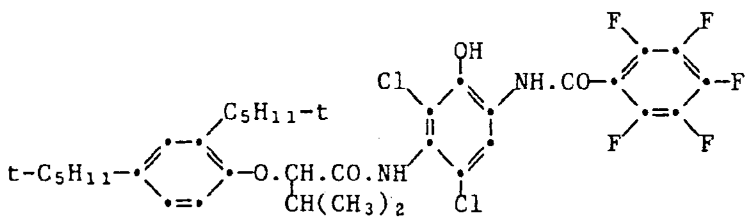

und die Menge eines der in den nachfolgenden Tabelle 6 und 7 angegebenen Lichtschutzmittel (resp. Lichtschutzmittelgemische) werden in 2 ml eines Gemisches von Dibutylphthalat/Aethylacetat (0,8 g/100 ml) gelöst. Zu 1 ml dieser Lösung gibt man 9 ml einer 2,3%igen wässrigen Gelatinelösung, die auf einen pH-Wert von 6,5 eingestellt ist und 0,872 g/l Netzmittel Nekal BX® (Diisobutylnaphthalin-sulfonsäure-Na-salz) enthält. Danach emulgiert man mit Ultraschall 3 Minuten.

Zu 5 ml der so erhaltenen Kuppleremulsion gibt man 2 ml einer Silberbromid-Emulsion mit einem Silbergehalt von 3 g pro Liter und 1 ml einer 0,7%igen wässrigen Lösung von Cyanursäuredichlorid als Gelatinehärter und vergiesst es auf ein 13 x 18 cm kunststoffbeschichtetes Papier. Nach einer Härtungszeit von 7 Tagen werden die Proben durch einen Silber-Stufenkeil mit 125 Lux·s belichtet und anschliessend im Ektaprint 2® -Prozess der Firma Kodak verarbeitet.

Die so erhaltenen Farbstufenkeile werden in einem Atlas Weather-Ometer® hinter einem UV-Filter (Kodak 2C) mit einer 2500 W Xenonlampe mit total 60 Kilojoule per $cm^2$ bestrahlt.

In den folgenden Tabellen 6 und 7 sind die dabei resultierenden prozentualen Farbdichteabnahmen bei einer ursprünglichen Farbdichte von 1,0 aufgeführt.

Tabelle 6

| Stabilisator | Menge (g) | Dichteverlust in % (hinter UV-Filter) 60 $KJ/cm^2$ |
|---|---|---|
| Keine | - | 35 |
| Thian A | 0,026 | 24 |
| Thian B | 0,026 | 24 |

Tabelle 7

| Stabilisator | Menge (g) | Dichteverlust in % (hinter UV-Filter) 60 $KJ/cm^2$ |
|---|---|---|
| Keine | - | 35 |
| Thian A | 0,026 | 24 |
| Thian A<br>+ Phenol 5 | 0,013<br>0,013 | 18 |
| Thian A | 0,026 | 24 |
| Thian A<br>+ Phenol 6 | 0,013<br>0,013 | 12 |

Beispiel IV:

0,031 g des Magenta-Kupplers der Formel

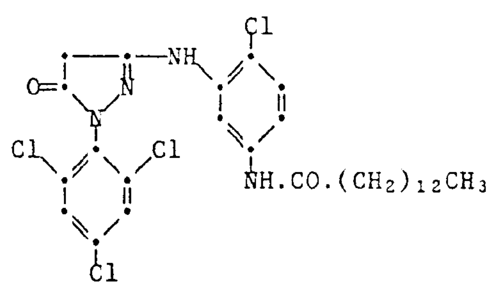

und die Menge eines der in der nachfolgenden Tabelle 8 angegebenen Stabilisatoren (resp. Stabilisatorengemische) werden in 2 ml eines Gemisches von Trikresylphosphat/Aethylacetat (0,769 g/100 ml) gelöst. Zu 1,0 ml dieser Lösung gibt man 9,0 ml einer 2,3 %igen wässrigen Gelatinelösung, die auf einen pH-Wert von 6,5 eingestellt ist und 0,436 g/lt Netzmittel Nekal BX® (Diisobutylnaphthalin-sulfonsäure-Na-salz) enthält. Danach emulgiert man mit Ultraschall 3 Minuten.

Zu 5,0 ml der so erhaltenen Kuppleremulsion gibt man 2 ml einer Silberbromid-Emulsion mit einem Silbergehalt von 6 g pro Liter, 1,0 ml einer 0,7 %igen wässrigen Lösung von Cyanursäuredichlorid als Gelatinehärter und vergiesst es auf ein 13x18 cm kunststoffbeschichtetes Papier. Nach einer Härtungszeit von 7 Tagen werden die Proben durch einen Silber-Stufenkeil mit 125 Lux.s belichtet und anschliessend im Ektaprint 2®-Prozess der Firma Kodak verarbeitet.

Die so erhaltenen Farbstufenkeile werden in einem Atlas Weather-Ometer® hinter einem UV-Filter (Kodak 2C) mit einer 3500 W Xenonlampe mit total 30 Kilojoule per $cm^2$ bestrahlt.

In der folgenden Tabelle 8 ist die Zunahme der Gelbfarbdichte im nicht belichteten Teil des Stufenkeils aufgeführt ($\Delta D_B$).

Tabelle 8

| Stabilisator | Menge (g) | $\Delta D_B$ |
|---|---|---|
| Keine | - | 16 % |
| Thian A | 0,011 | 6 % |
| Thian A | 0,0055 | 4 % |
| + Phenol 7 | 0,0055 | |
| Thian F | 0,011 | 7 % |
| Thian F | 0,0055 | 5 % |
| + Phenol 7 | 0,0055 | |

Thian F =

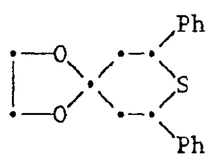

Phenol 7 =

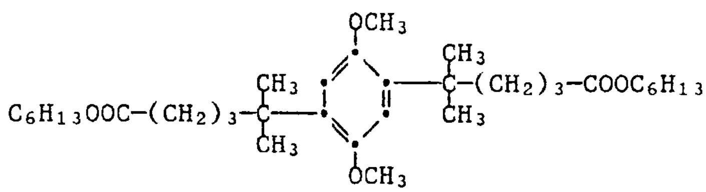

Beispiel V:

0,027 g des Magenta-Kupplers der Formel

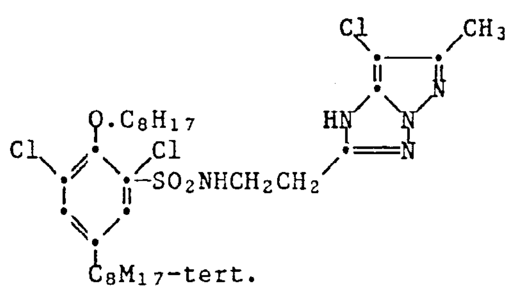

und die Menge eines der in der nachfolgenden Tabelle 9 angegebenen Stabilisatorengemische werden in 2 ml eines Gemisches von Trikresylphosphat/Aethylacetat (0,682 g/100 ml) gelöst. Zu 1,0 ml dieser Lösung gibt man 9,0 ml einer 2,3 %igen wässrigen Gelatinelösung, die auf einen pH-Wert von 6,5 eingestellt ist und 0,436 g/lt Netzmittel Nekal BX® (Diisobutylnaphthalin-sulfonsäure-Na-salz) enthält. Danach emulgiert man mit Ultraschall 3 Minuten.

Zu 5,0 ml der so erhaltenen Kunpleremulsion gibt man 2 ml einer Silberbromid-Emulsion mit einem Silbergehalt von 3 g pro Liter, 1,0 ml einer 0,7 %igen wässrigen Lösung von Cyanursäuredichlorid als Gelatinehärter und vergiesst es auf ein 13x18 cm kunststoffbeschichtetes Papier. Nach einer Härtungszeit von 7 Tagen werden die Proben durch einen Silber-Stufenkeil mit 125 Lux.s belichtet und anschliessend im Ektaprint 2®-Prozess der Firma Kodak verarbeitet.

Die so erhaltenen Farbstufenkeile werden in einem Atlas Weather-Ometer® hinter einem UV-Filter (Kodak

2C) mit einer 2500 W Xenonlampe mit total 30 Kilojoule per $cm^2$ bestrahlt.
In den folgenden Tabelle 9 ist die prozentuale Farbdichteabnahme aufgeführt.

Tabelle 9

| Stabilisator | Menge (g) | Dichteverlust in % (Remission) |
|---|---|---|
| Keine | - | 87 |
| Thian A<br>+ Phenol 7 | 0,008<br>0,008 | 29 |
| Thian A<br>+ Phenol 8 | 0,008<br>0,008 | 14 |
| Thian A<br>+ Phenol 9 | 0,008<br>0,008 | 22 |
| Thian A<br>+ Phenol 10 | 0,008<br>0,008 | 16 |
| Thian E<br>+ Phenol 7 | 0,008<br>0,008 | 26 |
| Thian E<br>+ Phenol 8 | 0,008<br>0,008 | 14 |
| Thian E<br>+ Phenol 9 | 0,008<br>0,008 | 22 |
| Thian E<br>+ Phenol 10 | 0,008<br>0,008 | 16 |
| Thian F<br>+ Phenol 7 | 0,008<br>0,008 | 30 |
| Thian F<br>+ Phenol 8 | 0,008<br>0,008 | 16 |
| Thian F<br>+ Phenol 9 | 0,008<br>0,008 | 21 |
| Thian F<br>+ Phenol 10 | 0,008<br>0,008 | 14 |

Phenol 8 =

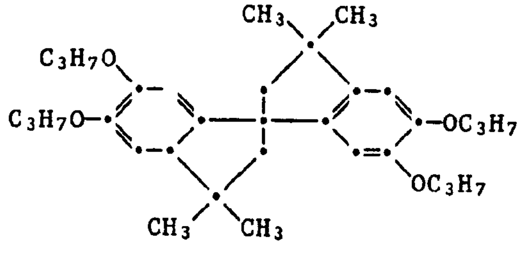

Phenol 9 = 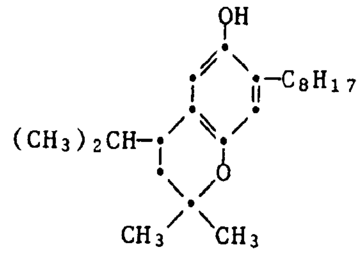

Phenol 10 = 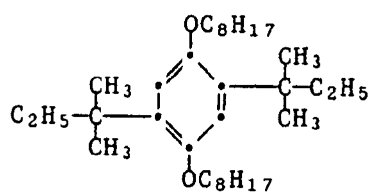

## Patentansprüche

1. Farbphotographisches Aufzeichnungsmaterial, enthaltend in mindestens einer Schicht als Stabilisator mindestens eine Tetrahydrothiopyranverbindung der Formel I

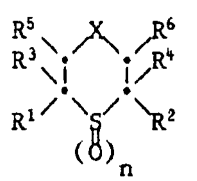 I

worin n 0, 1 oder 2 ist, $R^1$ und $R^2$ unabhängig voneinander Wasserstoff oder Methyl bedeuten, $R^3$ und $R^4$ unabhängig voneinander Wasserstoff, $C_1$-$C_4$-Alkyl, Phenyl, Thienyl oder durch 1 oder 2 $C_1$-$C_8$-Alkylgruppen, Cyclohexyl, Phenyl, $C_7$-$C_9$-Phenylalkyl, $C_1$-$C_{18}$-Alkoxy oder Halogen substituiertes Phenyl bedeuten, $R^5$ und $R^6$ unabhängig voneinander Wasserstoff, $C_1$-$C_4$-Alkyl, Phenyl, -COO($C_1$-$C_{18}$-Alkyl), -CO-$CH_3$, -CO-Phenyl, -CH($OR^7$)-$CH_3$ oder -CH($OR^7$)-Phenyl bedeuten und $R^7$ Wasserstoff, $C_1$-$C_{12}$-Alkyl, $C_2$-$C_{18}$-Alkanoyl oder Benzoyl bedeutet, X einen zweiwertigen Rest bedeutet, der den Ring der Formel I zu einem Tetrahydrothiopyranring ergänzt und der aus einer der folgenden Gruppen besteht:

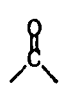 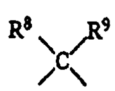 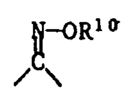 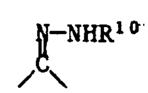 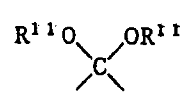

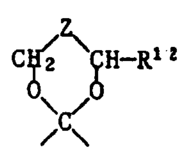 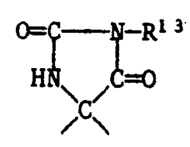 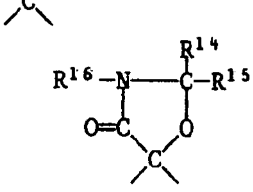

worin $R^8$ Wasserstoff, Methyl, Phenyl, -CN, -$CONH_2$, -COO($C_1$-$C_4$-Alkyl) oder -P(O)($OC_1$-$C_4$-Alkyl)$_2$ bedeutet, $R^9$ Wasserstoff, $OR^{17}$ oder -N($R^{18}$)($R^{19}$) bedeutet, $R^{10}$ Wasserstoff, $C_1$-$C_{18}$-Alkyl, $C_2$-$C_{18}$-Alkanoyl oder Benzoyl bedeutet, $R^{11}$ $C_1$-$C_{12}$-Alkyl, Allyl oder Benzyl bedeutet, $R^{12}$ Wasserstoff, $C_1$-$C_4$-Alkyl oder -$CH_2OR^{20}$ bedeutet, $R^{13}$ Wasserstoff, $C_1$-$C_{18}$-Alkyl, $C_7$-$C_9$-Aralkyl, Cyclohexyl oder Phenyl bedeutet, $R^{14}$ und $R^{15}$ unabhängig voneinander Wasserstoff, $C_1$-$C_{12}$-Alkyl oder Phenyl bedeuten oder $R^{14}$ und $R^{15}$ zusammen $C_4$-$C_{11}$-Alkylen bedeuten, $R^{16}$ Wasserstoff, $C_1$-$C_{12}$-Alkyl, durch -COO($C_1$-$C_4$-Alkyl) substituiertes $C_1$-$C_4$-Alkyl, Allyl oder Benzyl

bedeutet, $R^{17}$ Wasserstoff, $C_1$-$C_{12}$-Alkyl, Allyl, Benzyl, $C_2$-$C_{18}$-Alkanoyl, Benzoyl, eine Gruppe -CO-O-$R^{27}$ oder -CO-NH-$R^{27}$ oder eine Gruppe der Formel II oder III bedeutet,

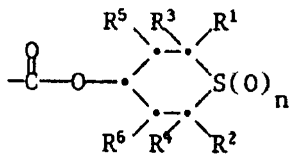

II

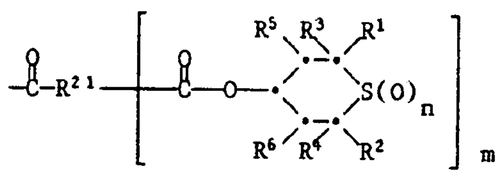

III

worin m 1, 2 oder 3 ist, $R^{18}$ Wasserstoff, $C_1$-$C_{12}$-Alkyl, $C_2$-$C_{18}$-Alkanoyl, Benzoyl, $C_1$-$C_{12}$-Alkoxycarbonyl, Phenoxycarbonyl oder Phenylaminocarbonyl bedeutet, $R^{19}$ Wasserstoff, $C_1$-$C_{12}$-Alkyl, Cyclohexyl, Benzyl, Phenyl oder eine Gruppe der Formel IV bedeutet,

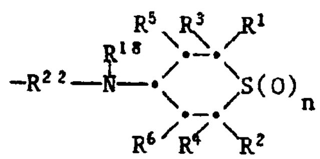

IV

$R^{20}$ Wasserstoff, $C_1$-$C_4$-Alkyl, $C_2$-$C_{18}$-Alkanoyl, Benzoyl oder eine Gruppe der Formel V oder VI

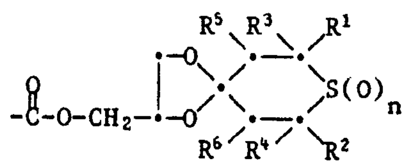

V

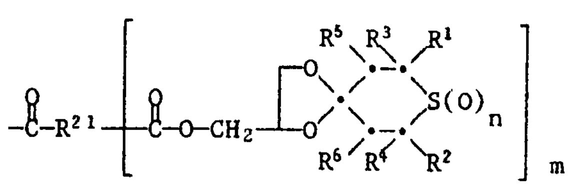

VI

bedeutet, worin m 1, 2 oder 3 ist, $R^{21}$, wenn m 1 ist, eine direkte Bindung, $C_1$-$C_{12}$-Alkylen, durch Phenyl oder Benzyl substituiertes oder durch -O- oder -S- unterbrochenes $C_2$-$C_6$-Alkylen, Vinylen, Phenylen oder eine Gruppe -NH-$R^{28}$-NH- bedeutet, wenn m 2 ist, $C_3$-$C_{12}$-Alkantriyl oder $C_6$-$C_{12}$-Arentriyl bedeutet, undwenn m 3 ist, $C_4$-$C_{12}$-Alkantetrayl oder $C_6$-$C_{12}$-Arentetrayl bedeutet, $R^{22}$ $C_2$-$C_{12}$-Alkylen, $C_4$-$C_8$-Alkenylen, Xylylen oder eine Gruppe -CO-$R^{23}$-CO- bedeutet, worin $R^{23}$ $C_1$-$C_{12}$-Alkylen, durch Phenyl oder Benzyl substituiertes oder durch -O- oder -S- unterbrochenes $C_2$-$C_6$-Alkylen, Vinylen oder Phenylen bedeutet, Z eine direkte Bindung, eine Gruppe

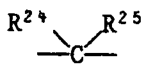

oder eine Gruppe der Formel

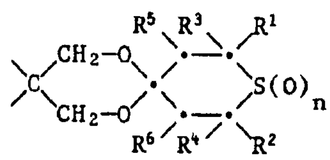

ist, worin $R^{24}$ Wasserstoff, $C_1$-$C_4$-Alkyl, -OH oder -$CH_2OR^{26}$ bedeutet, $R^{25}$ Wasserstoff oder $C_1$-$C_4$-Alkyl bedeutet und $R^{26}$ Wasserstoff, $C_1$-$C_4$-Alkyl, $C_2$-$C_{18}$-Alkanoyl, Benzoyl oder eine Gruppe der Formel VII oder VIII bedeutet,

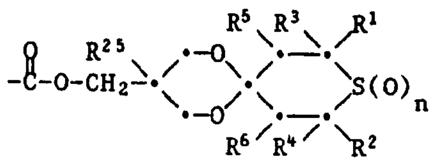

VII

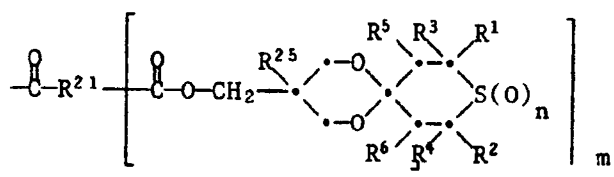

VIII

worin m 1, 2 oder 3 ist, $R^{27}$ $C_1$-$C_{12}$-Alkyl oder Phenyl bedeutet und $R^{28}$ $C_2$-$C_{12}$-Alkylen, $C_6$-$C_{12}$-Cycloalkylen, Phenylen, Naphthylen oder durch $C_1$-$C_4$-Alkyl, $C_1$-$C_4$-Alkoxy oder Halogen substituiertes Phenylen bedeutet.

2. Farbphotographisches Aufzeichnungsmaterial gemäss Anspruch 1, enthaltend mindestens eine Verbindung der Formel I, worin n 0 oder 2 ist, $R^1$ und $R^2$ Wasserstoff oder Methyl sind, $R^3$ und $R^4$ Methyl, Phenyl, Thienyl oder durch 1 oder 2 $C_1$-$C_4$-Alkylgruppen, durch Cyclohexyl, $C_1$-$C_4$-Alkoxy oder Chlor substituiertes Phenyl bedeuten, $R^5$ und $R^6$ unabhängig voneinander Wasserstoff, Methyl, Acetyl oder Benzoyl bedeuten, X eine der folgenden Gruppen bedeutet:

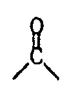 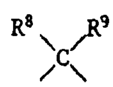 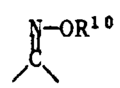 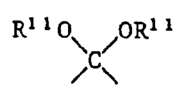 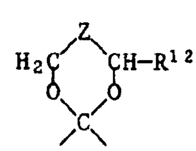

worin $R^8$ Wasserstoff,Methyl, Phenyl oder -P(O)(O$C_1$-$C_4$-Alkyl$)_2$ bedeutet, $R^9$ Wasserstoff, -$OR^{17}$ oder -$NHR^{18}$ bedeutet, $R^{10}$ Wasserstoff oder $C_2$-$C_{18}$-Alkanoyl ist, $R^{11}$ $C_1$-$C_4$-Alkyl ist, $R^{12}$ Wasserstoff, $C_1$-$C_4$-Alkyl oder -$CH_2OR^{20}$ bedeutet, Z eine direkte Bindung oder eine Gruppe -C($R^{24}$)($R^{25}$)- bedeutet, $R^{17}$ Wasserstoff, $C_2$-$C_{18}$-Alkanoyl, Benzoyl oder eine Gruppe der Formel III bedeutet, in der m 1 ist und $R^{21}$ $C_1$-$C_{12}$-Alkylen, Vinylen oder Phenylen ist, $R^{18}$ $C_2$-$C_{12}$-Alkanoyl oder Benzoyl bedeutet, $R^{20}$ Wasserstoff $C_2$-$C_{12}$-Alkanoyl, Benzoyl oder eine Gruppe der Formel VI bedeutet, in der m 1 ist, $R^{24}$ Wasserstoff, $C_1$-$C_4$-Alkyl oder -$CH_2OR^{26}$ bedeutet, $R^{25}$ Wasserstoff oder $C_1$-$C_4$-Alkyl bedeutet und $R^{26}$ Wasserstoff, $C_2$-$C_{12}$-Alkanoyl, Benzoyl oder eine Gruppe der Formel VIII bedeutet, in der m 1 ist.

3. Farbphotographisches Aufzeichnungsmaterial gemäss Anspruch 1, enthaltend eine Verbindung der Formel I, worin n 0 oder 2 ist, $R^1$ und $R^2$ Wasserstoff oder Methyl sind, $R^3$ und $R^4$ Methyl, Phenyl, Thienyl oder durch Methyl, Methoxy oder Chlor substituiertes Phenyl bedeuten, $R^5$ und $R^6$ unabhängig voneinander Was-

serstoff oder Acetyl bedeuten, X eine der folgenden Gruppen bedeutet:

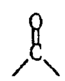
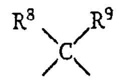
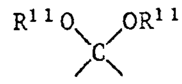
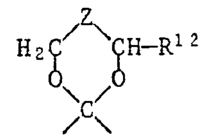

worin $R^8$ Wasserstoff, Methyl oder $-P(O)(OC_1-C_4\text{-Alkyl})_2$ bedeutet, $R^9$ $-OR^{17}$ ist, $R^{11}$ $C_1$-$C_4$-Alkyl bedeutet, $R^{12}$ Wasserstoff, $C_1$-$C_4$-Alkyl oder $-CH_2OH$ bedeutet, Z eine direkte Bindung oder eine Gruppe $-C(R^{24})(R^{25})$-ist, $R^{17}$ Wasserstoff, $C_2$-$C_{12}$-Alkanoyl, Benzoyl oder eine Gruppe der Formel III bedeutet, in der m 1 ist und $R^{21}$ $C_2$-$C_8$-Alkylen bedeutet, $R^{24}$ eine Gruppe $-CH_2OH$ und $R^{25}$ $C_1$-$C_4$-Alkyl bedeuten.

4. Farbphotographisches Aufzeichnungsmaterial gemäss Anspruch 1, enthaltend pro Schicht bis zu 1 g/m², vorzugsweise 10-300 mg/m² eines Stabilisators der Formel I.

5. Farbphotographisches Aufzeichnungsmaterial gemäss Anspruch 1, enthaltend

a) mindestens eine Tetrahydrothiopyranverbindung der Formel I und

b) mindestens ein phenolisches Antioxidans.

6. Farbphotographisches Aufzeichnungsmaterial gemäss Anspruch 5, worin die Komponente (b) ein Antioxidans ist, das mindestens eine Gruppe der Formel

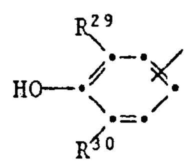

enthält, worin $R^{29}$ und $R^{30}$ unabhängig voneinander $C_1$-$C_{12}$-Alkyl, Cyclohexyl, Phenyl oder $C_7$-$C_9$-Phenylakyl bedeuten und $R^{30}$ auch Wasserstoff bedeuten kann.

7. Farbphotographisches Aufzeichnungsmaterial gemäss Anspruch 5, worin die Komponente (b) ein Antioxidans ist, das mindestens eine Gruppe der Formel

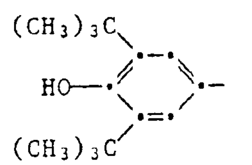

oder

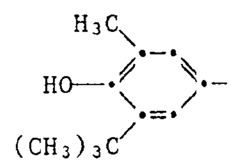

enthält.

8. Farbphotographisches Aufzeichnungsmaterial gemäss Anspruch 5, worin die Komponente (b) ein Alkylether eines sterisch gehinderten Phenols ist.

9. Farbphotographisches Aufzeichnungsmaterial gemäss Anspruch 5, enthaltend pro Schicht bis zu 1 g/m² des Gemisches von a) und b).

10. Farbphotographisches Aufzeichnungsmaterial gemäss Anspruch 5, enthaltend pro Schicht 10-300 mg/m² des Gemisches von a) und b).

11. Verbindungen der Formel Ia

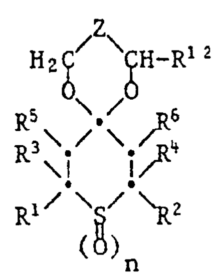

Ia

worin n 0, 1 oder 2 ist, $R^1$ und $R^2$ unabhängig voneinander Wasserstoff oder Methyl bedeuten, $R^3$ und $R^4$ unabhängig voneinander Wasserstoff, $C_1$-$C_4$-Alkyl, Phenyl, Thienyl oder durch 1 oder 2 $C_1$-$C_8$-Alkylgruppen, Cyclohexyl, Phenyl, $C_7$-$C_9$-Phenylalkyl, $C_1$-$C_{18}$-Alkoxy oder Halogen substituiertes Phenyl bedeuten, $R^5$ und $R^6$ unabhängig voneinander Wasserstoff, $C_1$-$C_4$-Alkyl, Phenyl, -COO($C_1$-$C_{18}$-Alkyl), -CO-$CH_3$, -CO-Phenyl, -CH($OR^7$)-$CH_3$ oder -CH($OR^7$)-Phenyl bedeuten und $R^7$ Wasserstoff, $C_1$-$C_{12}$-Alkyl, $C_2$-$C_{18}$-Alkanoyl oder Benzoyl bedeutet, $R^{12}$ Wasserstoff, $C_1$-$C_4$-Alkyl oder -$CH_2OR^{20}$ bedeutet, $R^{20}$ Wasserstoff, $C_1$-$C_4$-Alkyl, $C_2$-$C_{18}$-Alkanoyl, Benzoyl oder eine Gruppe der Formel V oder VI bedeutet,

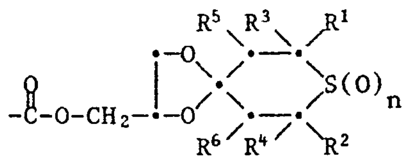

V

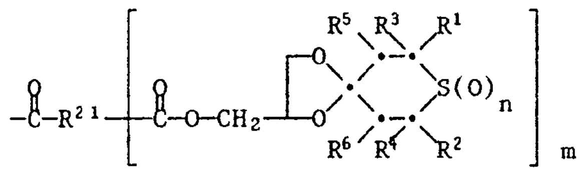

VI

bedeutet, worin m 1, 2 oder 3 ist, $R^{21}$, wenn m 1 ist, eine direkte Bindung, $C_1$-$C_{12}$-Alkylen, durch Phenyl oder Benzyl substituiertes oder durch -O- oder -S- unterbrochenes $C_2$-$C_6$-Alkylen, Vinylen, Phenylen oder eine Gruppe -NH-$R^{28}$-NH- bedeutet, wenn m 2 ist, $C_3$-$C_{12}$-Alkantriyl oder $C_6$-$C_{12}$-Arentriyl bedeutet, und wenn m 3 ist, $C_4$-$C_{12}$-Alkantetrayl oder $C_6$-$C_{12}$-Arentetrayl bedeutet, und $R^{28}$ $C_2$-$C_{12}$-Alkylen, $C_6$-$C_{12}$-Cycloalkylen, Phenylen, Naphthylen oder durch $C_1$-$C_4$-Alkyl, $C_1$-$C_4$-Alkoxy oder halogen substituiertes Phenylen bedeutet, und Z eine direkte Bindung oder eine Gruppe

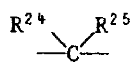

ist, worin $R^{24}$ Wasserstoff, $C_1$-$C_4$-Alkyl, -OH oder -$CH_2OR^{26}$ bedeutet, $R^{25}$ Wasserstoff oder $C_1$-$C_4$-Alkyl bedeutet und $R^{26}$ Wasserstoff, $C_1$-$C_4$-Alkyl, $C_2$-$C_{18}$-Alkanoyl, Benzoyl oder eine Gruppe der Formel VII oder VIII bedeutet,

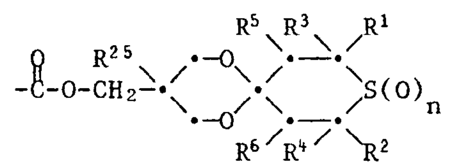

VII

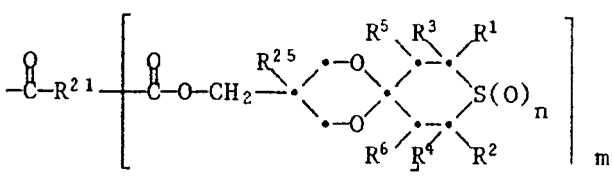

VIII

worin m 1, 2 oder 3 ist, ausgenommen die Verbindungen der Formeln

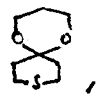, 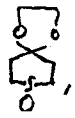,

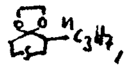, 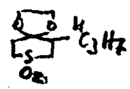 und 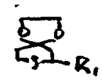

worin R Wassertoff Methyl, Alkyl, i-Propyl, n-Butyl oder Phenyl ist.

12. Verbindungen der Formel Ia gemäss Anspruch 11, worin n 0 oder 1 ist, $R^1$ und $R^2$ Wasserstoff oder Methyl sind, $R^3$ und $R^4$ Methyl, Phenyl, Thienyl oder durch 1 oder 2 $C_1$-$C_4$-Alkylgruppen, durch Cyclohexyl, $C_1$-$C_4$-Alkoxy oder Chlor substituiertes Phenyl bedeuten, $R^5$ und $R^6$ unabhängig voneinander Wasserstoff, Methyl, Acetyl oder Benzoyl bedeuten, $R^{12}$ Wasserstoff, $C_1$-$C_4$-Alkyl oder -$CH_2OR^{20}$ bedeutet, $R^{20}$ Wasserstoff, $C_2$-$C_{12}$-Alkanyol, Benzoyl oder eine Gruppe der Formel VI bedeutet, in der m 1 ist und $R^{21}$ $C_1$-$C_{12}$-Alkylen, Vinylen oder Phenylen ist, und Z eine direkte Bindung oder eine Gruppe -$C(R^{24})(R^{25})$- bedeutet, worin $R^{24}$ Wasserstoff, $C_1$-$C_4$-Alkyl oder -$CH_2OR^{26}$ bedeutet, $R^{25}$ Wasserstoff oder $C_1$-$C_4$-Alky bedeutet und $R^{26}$ Wasserstoff, $C_2$-$C_{12}$-Alkanoyl, Benzoyl oder eine Gruppe der Formel VIII bedeutet, in der m 1 ist.

13. Verbindungen der Formel Ia gemäss Anspruch 11, worin n 0 oder 2 ist, $R^1$ und $R^2$ Waserstoff oder Methyl sind, $R^3$ und $R^4$ Methyl, Phenyl, Thienyl oder durch Methyl, Methoxy oder Chlor substituiertes Phenyl bedeuten, $R^5$ und $R^6$ unabhängig voneinander Wasserstoff oder Acetyl bedeuten, $R^{12}$ Wasserstoff, $C_1$-$C_4$-Alkyl oder -$CH_2OH$ bedeutet, und Z eine direkte Bindung oder eine Gruppe -$C(R^{24})(R^{25})$- ist, worin $R^{24}$ eine Gruppe -$CH_2OH$ und $R^{25}$ $C_1$-$C_4$-Alkyl bedeutet.

14. Verwendung von Verbindungen des Anspruches 11 als Stabilisatoren für farbphotographische Materialien.

15. Verwendung gemäss Anspruch 14 zusammen mit einem phenolischen Antioxidans.

## Claims

1. A colour photography recording material containing in at least one layer, as a stabilizer, at least one tetrahydrothiopyran compound of the formula I

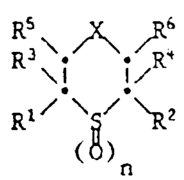

I

in which n is 0, 1 or 2, $R^1$ and $R^2$ independently of one another are hydrogen or methyl, $R^3$ and $R^4$ independently of one another are hydrogen, $C_1$-$C_4$alkyl, phenyl, thienyl or phenyl which is substituted by 1 or 2 $C_1$-$C_8$alkyl groups, cyclohexyl, phenyl, $C_7$-$C_9$phenylalkyl, $C_1$-$C_{18}$alkoxy or halogen, $R^5$ and $R^6$ independently of one another are hydrogen, $C_1$-$C_4$alkyl, phenyl, -$COO(C_1$-$C_{18}$alkyl), -$CO$-$CH_3$, -CO-phenyl, -$CH(OR^7)$-$CH_3$ or -$CH(OR^7)$-phenyl and $R^7$ is hydrogen, $C_1$-$C_{12}$alkyl, $C_2$-$C_{18}$alkanoyl or benzoyl, X is a divalent radical which completes the ring of the formula I to form a tetrahydrothiopyran ring and consists of one of the following groups:

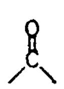 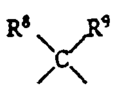 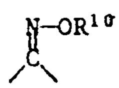 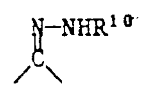 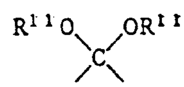

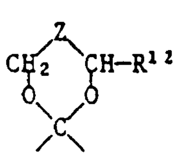 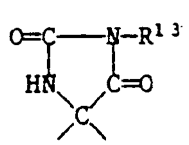 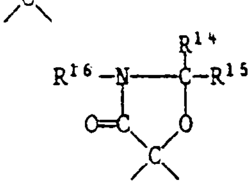

in which $R^8$ is hydrogen, methyl, phenyl, -CN, $-CONH_2$, -COO($C_1$-$C_4$alkyl) or -P(O)($OC_1$-$C_4$alkyl)$_2$, $R^9$ is hydrogen, $OR^{17}$ or -N($R^{18}$)($R^{19}$), $R^{10}$ is hydrogen, $C_1$-$C_{18}$alkyl, $C_2$-$C_{18}$alkanoyl or benzoyl, $R^{11}$ is $C_1$-$C_{12}$alkyl, allyl or benzyl, $R^{12}$ is hydrogen, $C_1$-$C_4$alkyl or $-CH_2OR^{20}$, $R^{13}$ is hydrogen, $C_1$-$C_{18}$alkyl, $C_7$-$C_9$aralkyl, cyclohexyl or phenyl, $R^{14}$ and $R^{15}$ independently of one another are hydrogen, $C_1$-$C_{12}$alkyl or phenyl or $R^{14}$ and $R^{15}$ together are $C_4$-$C_{11}$alkylene, $R^{16}$ is hydrogen, $C_1$-$C_{12}$alkyl, -COO($C_1$-$C_4$alkyl)-substituted $C_1$-$C_4$alkyl, allyl or benzyl, $R^{17}$ is hydrogen, $C_1$-$C_{12}$alkyl, allyl, benzyl, $C_2$-$C_{18}$alkanoyl, benzoyl, a group $-CO-O-R^{27}$ or $-CO-NH-R^{27}$ or a group of the formula II or III

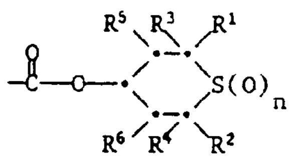

II

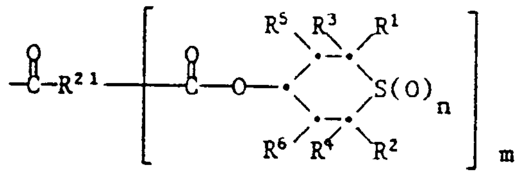

III

in which m is 1, 2 or 3, $R^{18}$ is hydrogen, $C_1$-$C_{12}$alkyl, $C_2$-$C_{18}$alkanoyl, benzoyl, $C_1$-$C_{12}$alkoxycarbonyl, phenoxycarbonyl or phenylaminocarbonyl, $R^{19}$ is hydrogen, $C_1$-$C_{12}$alkyl, cyclohexyl, benzyl, phenyl or a group of the formula IV

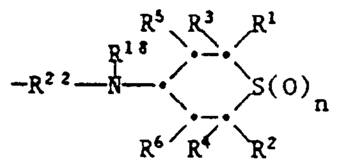

IV

$R^{20}$ is hydrogen, $C_1$-$C_4$alkyl, $C_2$-$C_{18}$alkanoyl, benzoyl or a group of the formula V or VI

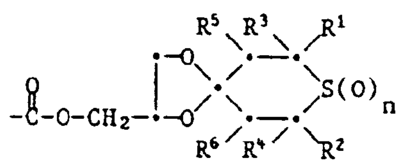

V

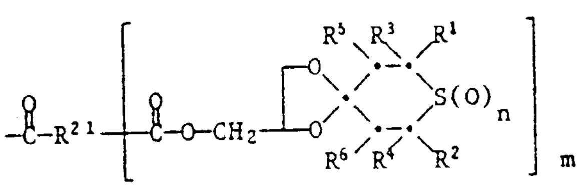

VI

in which m is 1, 2 or 3, $R^{21}$, if m is 1, is a direct bond, $C_1$-$C_{12}$alkylene, $C_2$-$C_6$alkylene which is substituted by phenyl or benzyl or interrupted by -O- or -S-, vinylene, phenylene or a group $-NH-R^{28}-NH-$, and if m is 2, is $C_3$-$C_{12}$alkanetriyl or $C_6$-$C_{12}$arenetriyl, and if m is 3, is $C_4$-$C_{12}$alkanetetrayl or $C_6$-$C_{12}$arenetetrayl, $R^{22}$ is $C_2$-$C_{12}$alkylene, $C_4$-$C_8$alkenylene, xylylene or a group $-CO-R^{23}-CO-$, in which $R^{23}$ is $C_1$-$C_{12}$alkylene which is substituted by phenyl or benzyl or interrupted by -O- or -S-, vinylene or phenylene, Z is a direct bond, a group

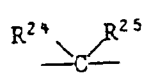

or a group of the formula

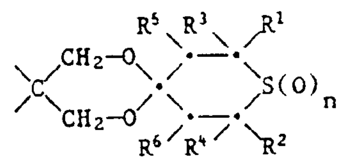

in which $R^{24}$ is hydrogen, $C_1$-$C_4$alkyl, -OH or -$CH_2OR^{26}$, $R^{25}$ is hydrogen or $C_1$-$C_4$alkyl and $R^{26}$ is hydrogen, $C_1$-$C_4$alkyl, $C_2$-$C_{18}$alkanoyl, benzoyl or a group of the formula VII or VIII

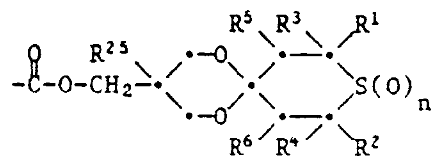

VII

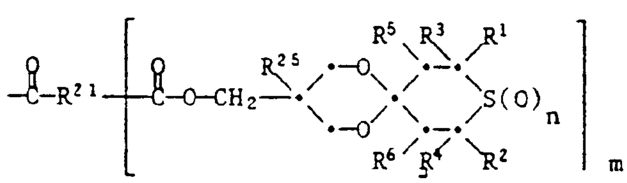

VIII

in which m is 1, 2 or 3, $R^{27}$ is $C_1$-$C_{12}$alkyl or phenyl and $R^{28}$ is $C_1$-$C_{12}$alkylene, $C_6$-$C_{12}$cycloalkylene, phenylene, naphthylene or phenyl which is substituted by $C_1$-$C_4$alkyl, $C_1$-$C_4$alkoxy or halogen.

2. A colour photography recording material according to claim 1, containing at least one compound of the formula I in which n is 0 or 2, $R^1$ and $R^2$ are hydrogen or methyl, $R^2$ and $R^3$ are methyl, phenyl, thienyl or phenyl which is substituted by 1 or 2 $C_1$-$C_4$alkyl groups, by cyclohexyl, $C_1$-$C_4$alkoxy or chlorine, $R^5$ and $R^6$ independently of one another are hydrogen, methyl, acetyl or benzoyl, X is one of the following groups:

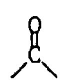 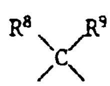 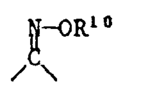 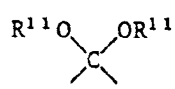 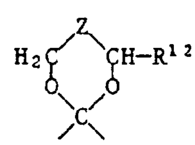

in which $R^8$ is hydrogen, methyl, phenyl or -P(O)($OC_1$-$C_4$alkyl$)_2$, $R^9$ is hydrogen, -$OR^{17}$ or -$NHR^{18}$, $R^{10}$ is hydrogen or $C_2$-$C_{18}$alkanoyl, $R^{11}$ is $C_1$-$C_4$alkyl, $R^{12}$ is hydrogen, $C_1$-$C_4$alkyl or -$CH_2OR^{20}$, Z is a direct bond or a group -C($R^{24}$)($R^{25}$)-, $R^{17}$ is hydrogen, $C_2$-$C_{18}$alkanoyl, benzoyl or a group of the formula III in which m is 1 and $R^{21}$ is $C_1$-$C_{12}$alkylene, vinylene or phenylene, $R^{18}$ is $C_2$-$C_{12}$alkanoyl or benzoyl, $R^{20}$ is hydrogen, $C_2$-$C_{12}$alkanoyl, benzoyl or a group of the formula VI in which m is 1, $R^{24}$ is hydrogen, $C_1$-$C_4$alkyl or -$CH_2OR^{26}$, $R^{25}$ is hydrogen or $C_1$-$C_4$alkyl and $R^{26}$ is hydrogen, $C_2$-$C_{12}$alkanoyl, benzoyl or a group of the formula VIII in which m is 1.

3. A colour photography recording material according to claim 1, containing a compound of the formula I in which n is 0 or 2, $R^1$ and $R^2$ are hydrogen or methyl, $R^3$ and $R^4$ are methyl, phenyl, thienyl or phenyl which is substituted by methyl, methoxy or chlorine, $R^5$ and $R^6$ independently of one another are hydrogen or acetyl, X is one of the following groups:

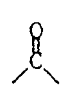 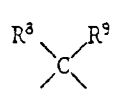 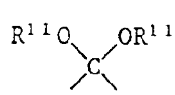 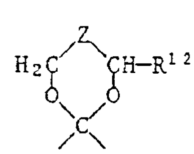

in which $R^8$ is hydrogen, methyl or -P( )($OC_1$-$C_4$alkyl$)_2$, $R^9$ is -$OR^{17}$, $R^{11}$ is $C_1$-$C_4$alkyl, $R^{12}$ is hydrogen, $C_1$-$C_4$alkyl or -$CH_2OH$, Z is a direct bond or a group -C($R^{24}$)($R^{25}$)-, $R^{17}$ is hydrogen, $C_2$-$C_{12}$alkanoyl, benzoyl or a group of

the formula III in which m is 1 and $R^{21}$ is $C_2$-$C_8$alkylene, $R^{24}$ is a group $-CH_2OH$ and $R^{25}$ is $C_1$-$C_4$alkyl.

4. A colour photography recording material according to claim 1, containing per layer up to 1 g/$m^2$, preferably 10-300 mg/$m^2$, of a stabilizer of the formula I.

5. A colour photography recording material according to claim 1, containing

a) at least one tetrahydrothiopyran compound of the formula I and

b) at least one phenolic antioxidant.

6. A colour photography recording material according to claim 5, in which component (b) is an antioxidant which contains at least one group of the formula

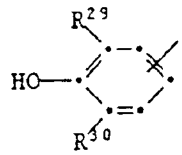

in which $R^{29}$ and $R^{30}$ independently of one another are $C_1$-$C_{12}$alkyl, cyclohexyl, phenyl or $C_7$-$C_9$phenylalkyl and $R^{30}$ can also be hydrogen.

7. A colour photography recording material according to claim 5, in which component (b) is an antioxidant which contains at least one group of the formula

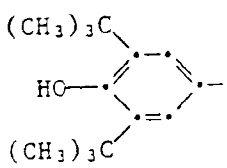

or

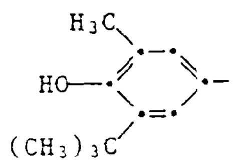

8. A colour photography recording material according to claim 5, in which component (b) is an alkyl ether of a sterically hindered phenol.

9. A colour photography recording material according to claim 5, containing per layer up to 1 g/$m^2$ of the mixture of (a) and (b).

10. A colour photography recording material according to claim 5, containing per layer 10-300 mg/$m^2$ of the mixture of (a) and (b).

11. A compound of the formula Ia

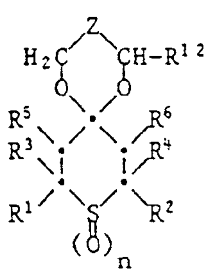

Ia

in which n is 0, 1 or 2, $R^1$ and $R^2$ independently of one another are hydrogen or methyl, $R^3$ and $R^4$ independently of one another are hydrogen, $C_1$-$C_4$alkyl, phenyl, thienyl or phenyl which is substituted by 1 or 2 $C_1$-$C_8$alkyl groups, cyclohexyl, phenyl, $C_7$-$C_9$phenylalkyl, $C_1$-$C_{18}$alkoxy or halogen, $R^5$ and $R^6$ independently of one another are hydrogen, $C_1$-$C_4$alkyl, phenyl, -COO($C_1$-$C_{18}$alkyl), $-CO-CH_3$, -CO-phenyl, $-CH(CR^7)-CH_3$ or $-CH(OR^7)$-phenyl and $R^7$ is hydrogen, $C_1$-$C_{12}$alkyl, $C_2$-$C_{18}$alkanoyl or benzoyl, $R^{12}$ is hydrogen, $C_1$-$C_4$alkyl or $-CH_2OR^{20}$, $R^{20}$ is hydrogen, $C_1$-$C_4$alkyl, $C_2$-$C_{18}$alkanoyl, benzoyl or a group of the formula V or VI

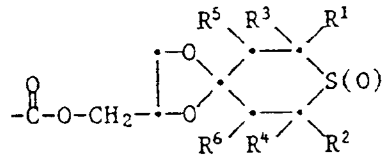

V

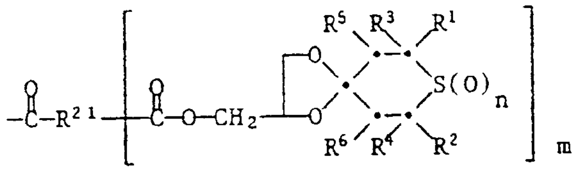

VI

in which m is 1, 2 or 3, $R^{21}$, if m is 1, is a direct bond, $C_1$-$C_{12}$alkylene, $C_2$-$C_6$alkylene which is substituted by phenyl or benzyl or interrupted by -O- or -S-, vinylene, phenylene or a group -NH-$R^{28}$-NH-, and if m is 2, is $C_3$-$C_{12}$alkanetriyl or $C_6$-$C_{12}$arenetriyl, and if m is 3, is $C_4$-$C_{12}$alkanetetrayl or $C_6$-$C_{12}$arenetetrayl, and $R^{28}$ is $C_2$-$C_{12}$alkylene, $C_6$-$C_{12}$cycloalkylene, phenylene, naphthylene or phenylene which is substituted by $C_1$-$C_4$alkyl, $C_1$-$C_4$alkoxy or halogen, Z is a direct bond or a group

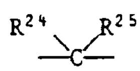 ,

in which $R^{24}$ is hydrogen, $C_1$-$C_4$alkyl, -OH or -$CH_2OR^{26}$, $R^{25}$ is hydrogen or $C_1$-$C_4$alkyl and $R^{26}$ is hydrogen, $C_1$-$C_4$alkyl, $C_2$-$C_{18}$alkanoyl, benzoyl or a group of the formula VII or VIII

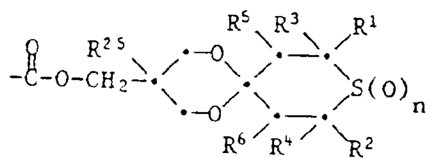

VII

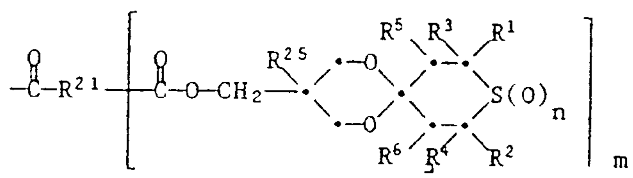

VIII

in which m is 1, 2 or 3, with the exception of a compound of the formulae

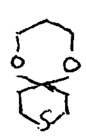 , 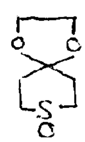 , 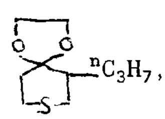 , 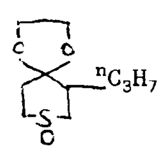 and 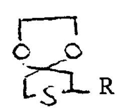 ,

in which R is hydrogen, methyl, ethyl, i-propyl, n-butyl or phenyl.

12. A compound of the formula Ia according to claim 11, in which n is 0 or 1, $R^1$ and $R^2$ are hydrogen or methyl, $R^3$ and $R^4$ are methyl, phenyl, thienyl or phenyl which is substituted by 1 or 2 $C_1$-$C_4$alkyl groups or by cyclohexyl, $C_1$-$C_4$alkoxy or chlorine, $R^5$ and $R^6$ independently of one another are hydrogen, methyl, acetyl or benzoyl, $R^{12}$ is hydrogen, $C_1$-$C_4$alkyl or -$CH_2OR^{20}$, $R^{20}$ is hydrogen, $C_2$-$C_{12}$alkanoyl, benzoyl or a group of the formula VI in which m is 1 and $R^{21}$ is $C_1$-$C_{12}$alkylene, vinylene or phenylene, and Z is a direct bond or a group -C($R^{24}$)($R^{25}$)-, in which $R^{24}$ is hydrogen, $C_1$-$C_4$alkyl or -$CH_2OR^{26}$, $R^{25}$ is hydrogen or $C_1$-$C_4$alkyl and $R^{26}$ is hyd-

rogen, $C_2$-$C_{12}$alkanoyl, benzoyl or a group of the formula VIII in which m is 1.

13. A compound of the formula I according to claim 11, in which n is 0 or 2, $R^1$ and $R^2$ are hydrogen or methyl, $R^3$ and $R^4$ are methyl, phenyl, thienyl or phenyl which is substituted by methyl, methoxy or chlorine, $R^5$ and $R^6$ independently of one another are hydrogen or acetyl, $R^{12}$ is hydrogen, $C_1$-$C_4$alkyl or -$CH_2OH$ and Z is a direct bond or a group -$C(R^{24})(R^{25})$-, in which $R^{24}$ is a group -$CH_2OH$ and $R^{25}$ is $C_1$-$C_4$alkyl.

14. The use of a compound of claim 11 as a stabilizer for colour photography materials.

15. Use according to claim 14 together with a phenolic antioxidant.

## Revendications

1. Matériau d'enregistrement photographique en couleur, contenant, dans au moins une couche, comme stabilisant, au moins un composé de tétrahydrothiopyranne de formule I :

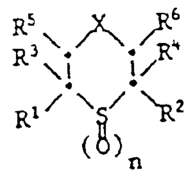

I

dans laquelle n vaut 0, 1 ou 2, $R^1$ et $R^2$ représentent chacun, indépendamment l'un de l'autre, un atome d'hydrogène ou un groupe méthyle, $R^3$ et $R^4$ représentent chacun, indépendamment l'un de l'autre, un atome d'hydrogène, un groupe alkyle en $C_1$ à $C_4$, phényle, thiényle ou un groupe phényle substitué par un ou deux groupes alkyles en $C_1$ à $C_8$ , par un groupe cyclohexyle, phényle, phénylalkyle en $C_7$ à $C_9$ , alcoxy en $C_1$ à $C_{18}$ ou halogéno, $R^5$ et $R^6$ représentent chacun, indépendamment l'un de l'autre, un atome d'hydrogène, un groupe alkyle en $C_1$ à $C_4$ phényle, -COO(alkyle en $C_1$ à $C_{18}$ ), -CO-$CH_3$,-CO-phényle, -CH($OR^7$)-$CH_3$ ou -CH($OR^7$)-phényle, et $R^7$ représente un atome d'hydrogène, un groupe alkyle en $C_1$ à $C_{12}$, alcanoyle en $C_2$ à $C_{18}$ ou benzoyle, X représente un reste divalent, qui complète le cycle de formule I pour donner un noyau ou cycle tétrahydrothiopyranne et qui consiste en l'un des groupes suivants :

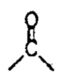
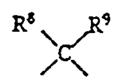
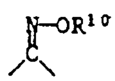
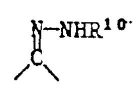
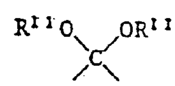

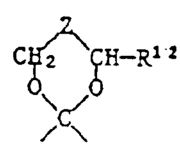
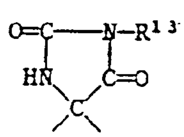
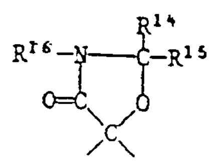

où $R^8$ représente un atome d'hydrogène, un groupe méthyle, phényle, -CN-$CONH_2$, -COO(alkyle en $C_1$ à $C_4$) ou -P(O)(O-alkyle en $C_1$ à $C_4$)$_2$ $R^9$ représente un atome d'hydrogène, un groupe $OR^{17}$ ou -N($R^{18}$) ($R^{19}$), $R^{10}$ représente un atome d'hydrogène, un groupe alkyle en $C_1$ à $C_{18}$, alcanoyle en $C_2$ à $C_{18}$ , ou benzoyle, $R^{11}$ représente un groupe alkyle en $C_1$ à $C_{12}$, allyle ou benzyle, $R^{12}$ représente un atome d'hydrogène, un groupe alkyle en $C_1$ à $C_4$, ou -$CH_2OH^{20}$ , $R^{13}$ représente un atome d'hydrogène, un groupe alkyle en $C_1$ à $C_{18}$, aralkyle en $C_7$ à $C_9$, cyclohexyle ou phényle, $R^{14}$ et $R^{15}$ représentent chacun, indépendamment l'un de l'autre, un atome d'hydrogène, un groupe alkyle en $C_1$ à $C_{12}$, ou phényle, ou bien $R^{14}$ et $R^{15}$ forment ensemble un groupe alkylène en $C_4$ à $C_{11}$, $R^{16}$ représente un atome d'hydrogène, un groupe alkyle en $C_1$ à $C_{12}$, un groupe alkyle en $C_1$ à $C_4$ (substitué par un groupe-COO (alkyle en $C_1$ à $C_4$)), allyle ou benzyle, $R^{17}$ représente un atome d'hydrogène, un groupe alkyle en $C_1$ à $C^{12}$, allyle, benzyle, alcanoyle en $C_2$ à $C_{-18}$, benzoyle, un groupe -CO-O-$R^{27}$ ou -CO-NH-$R^{27}$ ou un groupe de formule II ou III,

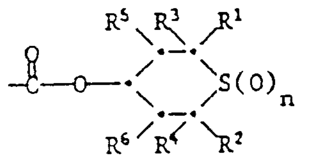

II

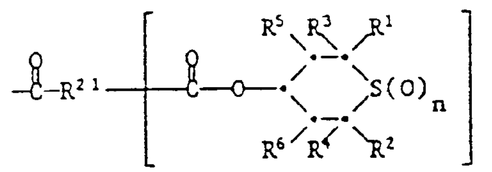

III

dans lesquelles m vaut 1,2,ou 3 $R^{18}$ représente un atome d'hydrogène, un groupe alkyle en $C_1$ à $C_{12}$, alcanoyle en $C_2$ à $C_{18}$ , benzoyle, alcoxycarbonyle en $C_1$ à $C_{12}$ , phénoxycarbonyle ou phénylaminocarbonyle, $R^{19}$ représente un atome d'hydrogène, un groupe alkyle en $C_1$ à $C_{12}$, cyclohexyle, benzyle, phényle ou un groupe de formule IV,

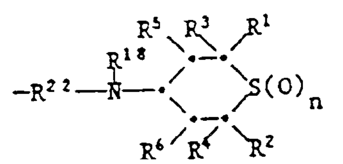

IV

$R^{20}$ représente un atome d'hydrogène, un groupe alkyle en $C_1$ à $C_4$, alcanoyle en $C_2$ à $C_{18}$ , benzoyle, ou un groupe de formule V ou VI,

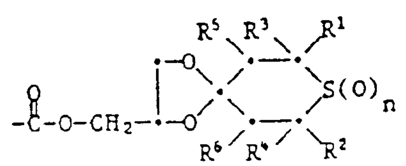

V

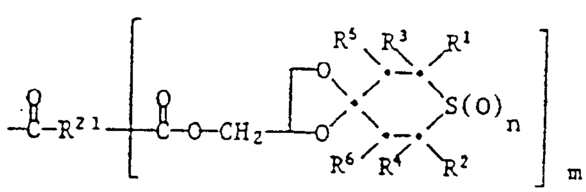

VI

où m vaut 1, 2, ou 3, $R^{21}$ représente : lorsque m vaut 1, une liaison directe, un groupe alkylène en $C_1$ à $C_{12}$ , un groupe alkylène en $C_2$ à $C_6$ (substitué par un groupe phényle ou benzyle ou interrompu par -O- ou -S-), un groupe vinylène, phénylène ou un groupe -NH-$R^{28}$-NH-, Lorsque m vaut 2, $R^{21}$ représente un groupe alcane triyle en $C_3$ à $C_{12}$, ou arène triyle en $C_6$ à $C_{12}$, et , lorsque m vaut 3, $R^{21}$ représente un groupe alcane tétrayle en $C_4$ à $C_{12}$ ou un arène tétrayle en $C_6$ à $C_{12}$, $R^{22}$ représente un groupe alkylène en $C_2$ à $C_{12}$, alcénylène en $C_4$ à $C_8$, xylylène ou un groupe -CO-$R^{23}$-CO-, où $R^{23}$ représente un groupe alkylène en $C_1$ à $C_{12}$, un groupe alkylène en $C_2$ à $C_6$ (substitué par un groupe phényle ou benzyle ou interrompu par -O- ou -S-), un groupe vinylène ou phénylène, Z représente une liaison directe, un groupe

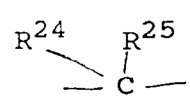

ou un groupe de formule :

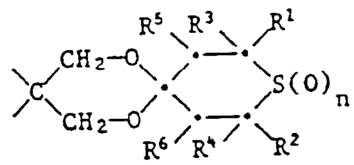

$R^{24}$ représente un atome d'hydrogène, un groupe alkyle en $C_1$ à $C_4$-OH, ou - $CH_2OR^{26}$, $R^{25}$ représente un atome d'hydrogène ou un groupe alkyle en $C_1$ à $C_4$, et $R^{26}$ représente un atome d'hydrogène, un groupe alkyle en $C_1$ à $C_4$, alcanoyle en $C_2$ à $C_{18}$ , benzoyle ou un groupe de formule VII ou VIII

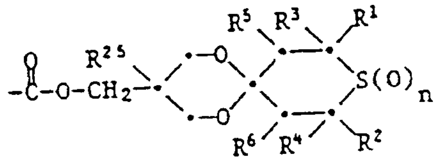

VII

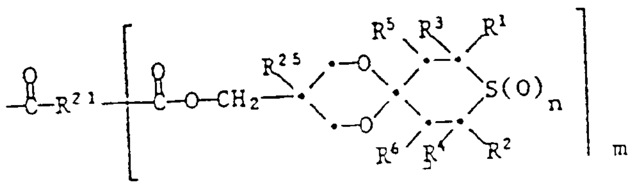

VIII

dans lesquelles m vaut 1, 2 ou 3, $R^{27}$ représente un groupe alkyle en $C_1$ à $C_{12}$ ou phényle et , $R^{28}$ représente un groupe alkylène en $C_2$ à $C_{12}$, cycloalkylène en $C_6$ à $C_{12}$, phénylène, napthylène ou un groupe phénylène subtitué par un groupe alkyle en $C_1$ à $C_4$, alcoxy en $C_1$ à $C_4$ ou halogéno.

2. Matériau pour enregistrement photographique en couleur selon la revendication 1, contenant au moins un composé de formule I, dans lequel m vaut 0 ou 2, $R^1$ et $R^2$ représentent chacun un atome d'hydrogène ou un groupe méthyle, $R^3$ et $R^4$ représentent chacun un groupe méthyle, phényle, thiényle ou un groupe phényle (substitué par un ou deux groupes alkyles en $C_1$ à $C_4$, par un groupe cyclohexyle, alcoxy en $C_1$ à $C_4$, ou par du chlore), $R^5$ et $R^6$ représentent chacun, indépendamment l'un de l'autre un atome d'hydrogène, un groupe méthyle, acétyle ou benzoyle, X représente l'un des groupes suivants :

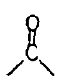 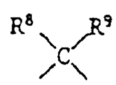 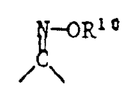 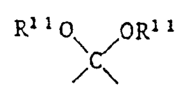 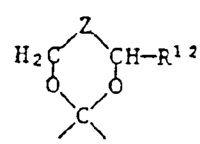

où $R^8$ représente un atome d'hydrogène, un groupe méthyle, phényle ou -P(O)(O alkyle en $C_1$ à $C_4)_2$, $R^9$ représente un atome d'hydrogène, un groupeOR$^{17}$ ou -NHR$^{18}$, $R^{10}$ représente un atome d'hydrogène ou un groupe alcanoyle en $C_2$ à $C_{18}$ , $R^{11}$ représente un groupe alkyle en $C_1$ à $C_4$, $R^{12}$ représente un atome d'hydrogène, un groupe alkyle en $C_1$ à $C_4$, ou -$CH_2OR^{20}$ Z est une liaison directe ou représente un groupe -C($R^{24}$)($R^{25}$)-, $R^{17}$ représente un atome d'hydrogène, un groupe alcanoyle en $C_2$ à $C_{18}$, benzoyle ou un groupe de formule II, dans laquelle m vaut 1, et $R^{21}$ représente un groupe alkylène en $C_1$ à $C_{12}$, vinylène ou phénylène, $R^{18}$ représente un groupe alcanoyle en $C_2$ à $C_{12}$ ou benzoyle, $R^{20}$ représente un atome d'hydrogène, un groupe alcanoyle en $C_2$ à $C_{12}$, benzoyle ou un groupe de formule VI dans laquelle m vaut 1, $R^{24}$ représente un atome d'hydrogène, un groupe alkyle en $C_1$ à $C_4$ ou -$CH_2OR^{26}$, $R^{25}$ représente un atome d'hydrogène ou un groupe alkyle en $C_1$ à $C_4$ et, $R^{26}$ représente un atome d'hydrogène, un groupe alcanoyle en $C_2$ à $C_{12}$, benzoyle ou un groupe de formule VIII, dans laquelle m vaut 1,

3. Matériau pour enregistrement photographique en couleur selon la revendication 1, contenant un composé de formule I, dans laquelle m est nul ou vaut 2, $R^1$ et $R^2$ représentent chacun un atome d'hydrogène ou un groupe méthyle, $R^3$ et $R^4$ représentent chacun un groupe méthyle, phényle, thiényle ou un groupe phényle substitué pwar un groupe méthyle, méthoxy ou chloro, $R^5$ et $R^6$ représentent chacun, indépendamment l'un de l'autre, un atome d'hydrogène ou un groupe acétyle, X représente l'un des groupes suivants:

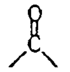 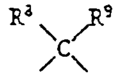 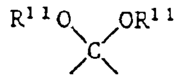 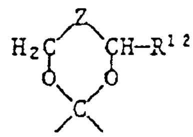

où $R^8$ représente un atome d'hydrogène, un groupe méthyle ou -P(O)(O-alkyle en $C_1$ à $C_4)_2$, $R_9$ représente -$OR_{17}$, $R^{11}$ représente un groupe alkyle en $C_1$ à $C_4$, $R^{12}$ représente un atome d'hydrogène, un groupe alkyle en $C_1$ à $C_4$ ou -$CH_2OH$ , Z est une liaison directe ou un groupe -C($R^{24}$)($R^{25}$)-, $R^{17}$ représente un atome d'hydrogène, un groupe alcanoyle en $C_2$ à $C_{12}$, benzoyle ou un groupe de formule III, dans laquelle m vaut 1 et $R^{21}$ représente un groupe alkylène en $C_2$ à $C_8$, $R^{24}$ représente un groupe $CH_2OH$ et $R^{25}$ représente un groupe alkyle en $C_1$ à $C_4$.

4. Matériau pour enregistrement photographique en couleur selon la revendication 1, contenant par couche jusqu'à 1 g/m², avantageusement 10 à 300 mg/m² d'un stabilisant répondant à la formule I,

5. Matériau pour enregistrement photographique en couleur selon la revendication I, contenant :

a) au moins un tétrahydrothiopyranne de formule I, et

b) au moins un anti-oxydant phénolique,

6. Matériau pour enregistrement photographique en couleur selon la revendication 5, dans lequel le composant (b) est un anti-oxydant contenant au moins un groupe de formule

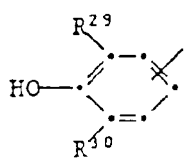

dans laquelle $R^{29}$ et $R^{30}$ représentent chacun indépendamment l'un de l'autre, un groupe alkyle en $C_1$ à $C_{12}$, cyclohexyle, phényle ou phénylalkyle en $C_7$ à $C_9$, et $R^{30}$ peut également représenter un atome d'hydrogène.

7. Matériau pour enregistrement photographique en couleur selon la revendication 5, dans lequel le composant (b) est un anti-oxydant qui contient au moins un groupe de formule

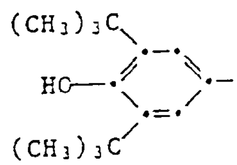 ou 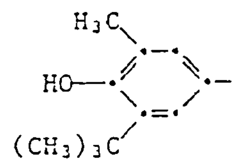

8. Matériau pour enregistrement photographique en couleur selon la revendication 5, dans lequel le composant (b) est un éther alkylique d'un phénol présentant de l'empêchement stérique.

9. Matériau pour enregistrement photographique en couleur selon la revendication 5, contenant par couche jusqu'à 1 m g/m² du mélange de (a) et (b).

10. Matériau pour enregistrement photographique en couleur selon la revendication 5, contenant par couche 10 à 300 mg/m² du mélange de (a) et de (b) .

11. Composés de formule Ia :

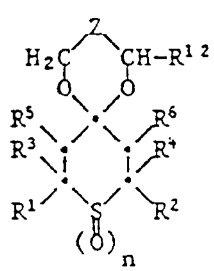

Ia

dans laquelle n vaut 0, 1 ou 2, $R^1$ et $R^2$ représentent chacun, indépendamment l'un de l'autre, un atome d'hydrogène ou un groupe méthyle, $R^3$ et $R^4$ représentent chacun, indépendamment l'un de l'autre, un atome d'hydrogène, un groupe alkyle en $C_1$ à $C_4$, phényle, thiényle ou un groupe phényle substitué par un ou deux groupes alkyles en $C_1$ à $C_8$, cyclohexyle, phényle, phénylalkyle en $C_7$ à $C_9$, alcoxy en $C_1$ à $C_{18}$ ou halogéno, $R^5$ et $R^6$ représentent chacun, indépendamment l'un de l'autre, un atome d'hydrogène, un groupe alkyle en $C_1$ à $C_4$, phényle, -COO(alkyle en $C_1$ à $C_{-18}$), $-CO-CH_3$, -CO-phényle, $-CH(OR^7)-CH_3$ ou $-CH(OR^7)$-phényle, et $R^7$ représente un atome d'hydrogène, un, groupe alkyle en $C_1$ à $C_{12}$, alcanoyle en $C_2$ à $C_{18}$ ou benzoyle, $R^{12}$ représente un atome d'hydrogène, un groupe alkyle en $C_1$ à $C_4$ ou $-CH_2OR^{20}$, $R^{20}$ représente un atome d'hydrogène, un groupe alkyle en $C_1$ à $C_4$, alcanoyle en $C_2$ à $C_{18}$, benzoyle ou un groupe de formule V ou VI,

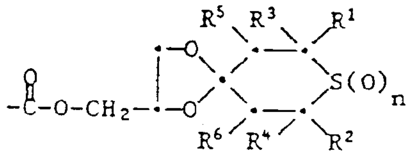

V

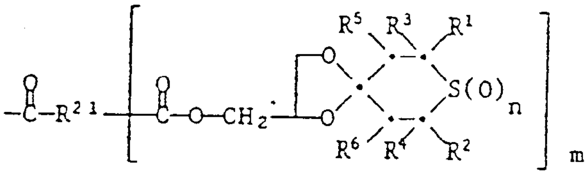

VI

dans lesquelles m vaut 1, 2 ou 3, $R^{21}$ représente, lorsque m vaut 1, une liaison directe, un groupe alkylène en $C_1$ à $C_{12}$, un groupe alkylène en $C_2$ à $C_6$ (substitué par un groupe phényle ou benzyle ou interrompu par -O- ou -S-), un groupe vinylène, phénylène ou un groupe $NH-R^{28}-NH$, Lorsque m vaut 2, $R^{21}$ représente un groupe alcanetriyle en $C_3$ à $C_{12}$ ou arène triyle en $C_6$ à $C_{12}$, et lorsque m vaut 3, $R^{21}$ représente un groupe alcanetétrayle en $C_4$ à $C_{12}$ ou arène tétrayle en $C_6$ à $C_{12}$, et $R^{28}$ représente un groupe alkylène en $C_2$ à $C_{12}$, cycloalkylène en $C_6$ à $C_{12}$, phénylène, naphtylène, ou un groupe phénylène substitué par un groupe alkyle en $C_1$ à $C_4$, alcoxy en $C_1$ à $C_4$, ou halogéno, Z représente une liaison directe ou un groupe

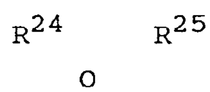

$R^{24}$ représente un atome d'hydrogène , un groupe alkyle en $C_1$ à $C_4$, -OH ou $-CH_2OR^{26}$, $R^{25}$ représente un atome d'hydrogène ou un groupe alkyle en $C_1$ à $C_4$, et $R^{26}$ représente un atome d'hydrogène, un groupe alkyle en $C_1$ à $C_4$, alcanoyle en $C_2$ à $C_{18}$, benzoyle ou un groupe de formule VII ou VIII,

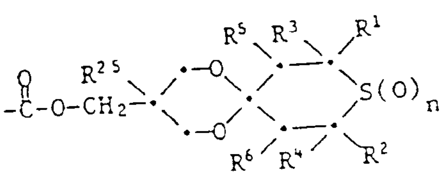

VII

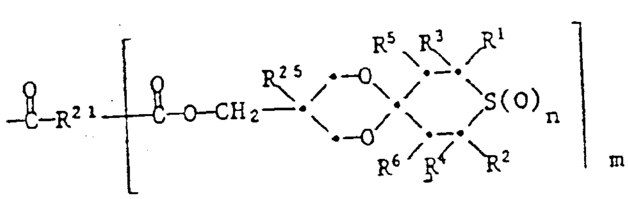

VIII

(dans lesquelles m vaut 1, 2 ou 3), à l'exclusion des composés de formule

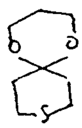 , 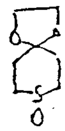 , 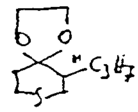 ,

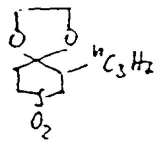 et 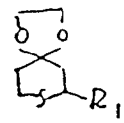

dans lesquelles R représente un atome d'hydrogène, un groupe méthyle, éthyle, isopropyle, butyle ou phényle.

12. Composés de formule Ia selon la revendication 11, dans lesquels n vaut 0 ou 1, $R^1$ et $R^2$ représentent chacun atome d'hydrogène ou un groupe méthyle, $R^3$ et $R^4$ représentent chacun un groupe méthyle, phényle, thiényle ou un groupe phényle (substitué par un ou deux groupes alkyles en $C_1$ à $C_4$, par un groupe cyclohexyle, alcoxy en $C_1$ à $C_4$, ou chloro), $R^5$ et $R^6$ représentent chacun, indépendamment l'un de l'autre, un atome d'hydrogène, un groupe méthyle, acétyle ou benzoyle; $R^{12}$ représente un atome d'hydrogène, un groupe alkyle en $C_1$ à $C_4$, ou $-CH_2OR^{20}$, $R^{20}$ représentent un atome d'hydrogène, un groupe alcanoyle en $C_2$ à $C_{12}$, benzoyle ou un groupe de formule VI, dans laquelle m vaut 1 et $R^{21}$ représente un groupe alkylène en $C_1$ à $C_{12}$, vinylène ou phénylène, et Z est une liaison directe ou représente un groupe $-C(R^{24})(R^{25})-$, et $R^{24}$ représente un atome d'hydrogène, un groupe alkyle en $C_1$ à $C_4$, ou $-CH_2OR^{26}$, $R^{25}$ représente un atome d'hydrogène ou un groupe alkyle en $C_1$ à $C_4$, et $R^{26}$ représente un atome d'hydrogène, un groupe alcanoyle en $C_2$ à $C_{12}$, benzoyle ou un groupe de formule VIII, dans laquelle m vaut 1,

13. C omposés de formule 1A selon la revendication 11, dans laquelle n vaut 0 ou 2, $R^1$ et $R^2$ représentent chacun un atome d'hydrogène ou un groupe méthyle, $R^3$ et $R^4$ représentent chacun un groupe méthyle, phényle, thiényle ou un groupe phényle (substitué par un groupe méthyle, méthoxy ou chloro), $R^5$ et $R^6$ représentent chacun, indépendamment l'un de l'autre, un atome d'hydrogène ou un groupe acétyle ; $R^{12}$ représente un atome d'hydrogène, un groupe alkyle en $C_1$ à $C_4$ ou $-CH_2OH$, et Z est une liaison directe ou représente un groupe $-C(R^{24})$ $(R^{25})-$, où $R^{24}$ représente un groupe $-CH_2OH$ et $R^{25}$ représente un groupe alkyle en $C_1$ à $C_4$.

14. Utilisation de composés selon la revendication 11 comme stabilisants pour des matériaux pour photographie en couleur.

15. Utilisation selon la revendication 14 effectuée en association avec un anti-oxydant phénolique.